# EUROPEAN PATENT SPECIFICATION

(11) **EP 1 348 762 B1**
(45) Date of publication and mention of the grant of the patent: **27.01.2010**
(21) Application number: 01270606.5
(22) Date of filing: 12.12.2001
(51) Int. Cl.: C12Q 1/68

(54) **METHOD OF ANALYZING GENE EXPRESSION**
VERFAHREN ZUR GENEXPRESSIONSANALYSE
METHODE D'ANALYSE D'EXPRESSION GENIQUE

(30) Priority: 12.12.2000 JP 2000377887
(43) Date of publication of application: 01.10.2003
(73) Proprietor: National Institute of Radiological Sciences, Chiba-shi, Chiba 263-8555 (JP); Maze, Inc, Tokyo 151-0072 (JP); Abe, Masumi, Chiba-shi, Chiba 263-0025 (JP); MessengerScape Co. Ltd., Shibuya-ku Tokyo 151-0072 (JP)
(72) Inventor: ABE, Masumi, Chiba-shi, Chiba 263-0025 (JP); SAITO, Toshiyuki, Funabashi-shi, Chiba 273-0003 (JP); HATTORI, Atsushi, Kisarazu-shi, Chiba 292-0044 (JP); SATO, Shinji, Isumi-gun, Chiba 299-4504 (JP); KASAMA, Yasuji, Cyosei-gun, Chiba 299-4333 (JP)
(74) Representative: Schmid, Wolfgang
(86) International application number: PCT/JP2001/010898
(87) International publication number: WO 2002/048352

(56) References cited:
- WO-A-00/08208
- WO-A-98/51789
- WO-A-98/58083
- WO-A2-98/35058
- WO-A2-98/51789
- JP-A- 11 196 874
- JP-A- 2001 037 487
- JP-A- 2001 299 394
- US-A- 5 824 516
- MADDEN S.L. ET AL.: "Serial Analysis of Gene Expression: From gene discovery to target identification" DRUG DISCOVERY TODAY, vol. 5, no. 9, September 2000 (2000-09), pages 415-425, XP002328839 ISSN: 1359-6446
- KATO, K.: 'Adaptor-tagged competitive PCR: a novel method for measuring relative gene expression' NUCLEIC ACIDS RES. vol. 25, no. 22, 1997, pages 4694 - 4696, XP002900285
- MATOBA, R. ET AL.: 'Gene expression profiling of mouse postnatal cerebellar development' PHYSIOL GENOMICS vol. 4, no. 2, 18 December 2000, pages 155 - 164, XP002909423

## Description

The present invention is based on Japanese Patent Application No. 2000-377887 filed on December 12, 2000 and claims the priority thereof.

### Technical Field

The present invention relates to a method of producing an expression profile of gene and a method of analyzing expression of gene.

### Background Art

In 2000, determination of the whole sequence of the human genome approached completion. The enormous amount of information obtained as a result of the determination will be the base for comprehensively understanding a network including all of the genes and gene products thereof expressed in specific cells.

Examples of the method employed as a means for analyzing such a network include: the differential display method disclosed in USP 5,262,311 and USP 5,599,672; the serial analysis of gene expression (which will be referred to as "SAGE" hereinafter) disclosed in Japanese Patent Application KOHYO No. 10-511002; and the method of using a micro-array and a DNA chip disclosed in USP 5,807,522, USP 5,700,637 and USP 5,744,305. WO 98/51789 A2 describes a method to clone mRNAs and display of differentially expressed transcripts (DODET). In this method, the expression patterns in cells, especially in eukaryotic cells, are investigated.

The differential display method is a method in which cDNA prepared from a cell is used as a substrate. By carrying out PCR for the cDNA prepared from a cell, by using a plurality of types of anchor primer and an optional primer, various types of gene expression in a cell can optionally be analyzed. However, according to this method, only a portion of the whole genes can be analyzed. Further, use of an anchor primer and an optional primer results in poor reproducibility, which is problematic.

On the contrary, SAGE is a method which enables obtaining an expression profile for all of the genes expressed in a cell. In SAGE, analysis is carried out by using cDNA which has been prepared by using mRNA prepared from a cell. The method includes: a step of treating the prepared cDNA with a restriction enzyme; a step of cutting out fragments of approximately 9 to 11 base pairs; a step of ligating the fragments derived from the obtained cDNA of various types; and effecting sequencing. However, in SAGE, sequencing has to be carried out approximately 100,000 times in order to obtain the information on approximately 50% of all the types of the expressed genes. In short, SAGE is very costly. Further, in the case of SAGE, the fragments derived from cDNA are generally short. In actual practice, separation of genes in the form of such short fragments is often impossible.

The micro-array of USP 5,807,522 and the DNA chip of USP 5,700,637 and USP 5,744,305 are produced by fixing a probe of a known gene on a solid phase. In the methods using such a micro-array and DNA chip, an expression profile of a gene is obtained by hybridizing a sample with the probe. In these methods, the sequence of the gene to be detected must be already known.

### Disclosure of Invention

A first object of the present invention is to provide a method which enables producing a wide-range gene expression profile (i.e., an expression profile of variety of types of genes). A second object of the present invention is to provide a method of analyzing gene expression of a variety of types.

The above-mentioned first object is achieved by a method of producing a gene expression profile, comprising:
(a) a step of synthesizing cDNA from mRNA extracted from a cell, such that a tag substance is added to the 5' terminal of the cDNA;
(b) a step of cutting the product obtained as a result of the reaction in step (a) with a first restriction enzyme X;
(c) a step of connecting, to a fragment obtained in step (b), an "X" adaptor having a sequence complementary to a sequence of a site of the fragment at which site the incision with the first restriction enzyme X has been effected;
(d) a step of connecting the fragment obtained in step (c) to a substance having high affinity with respect to the tag substance, thereby collecting the fragment;
(e) a step of cutting the fragment collected in step (d) with a second restriction enzyme Y and removing a fragment connected to the tag substance, thereby obtaining a fragment including the 5' side-portion of the cut cDNA;
(f) a step of adding, to a fragment obtained in step (e), a "Y" adaptor having a sequence complementary to a sequence of a site of the fragment at which site the incision with the second restriction enzyme Y has been effected;
(g) a step of carrying out a PCR reaction, for the fragment obtained in step (f), by using a primer which has a sequence complementary to the sequence of the "X" adaptor and two-nucleotide sequence (NN) at the 3' terminal thereof, and a primer which has a sequence complementary to the sequence of the "Y" adaptor and two-nucleotide sequence (NN) at the 3' terminal thereof; and
(h) a step of subjecting the obtained PCR product to electrophoresis and detecting a migration distance and a peak, thereby producing gene an expression profile.

The second object is achieved by a method of analyzing gene expression, comprising:
(a) a step of producing a gene expression profile, for each of a control cell and a subject cell, by employing the above-mentioned method of producing a gene expression profile; and
(b) a step of analyzing a change in gene expression at the subject cell, by comparing the two profiles of gene expression obtained in step (a).

Other objects and advantages of the present invention will be described by the description and examples hereinafter. The present invention will more clearly be understood with reference to these description and examples. Further, the objects and advantages of the present invention will be understood in detail and achieved by means of the methods and combination thereof described below.

### Brief Description of Drawings

The accompanying drawings, which are incorporated into the present specification and constitute a portion thereof, naturally describe the concept of the preferred embodiment, the aforementioned general description and the details of the preferred embodiment described below, of the present invention. The drawings are also used for describing the fundamental idea of the present invention.
FIG. 1 is a view which schematically shows a method of producing a gene expression profile according to an embodiment of the present invention.
FIG. 2 is a scheme which shows a method of producing a gene expression profile according to the embodiment of the present invention.
FIG. 3 is one example of a chart showing a portion of the gene expression profile obtained according to the embodiment of the present invention.
FIG. 4 is a table which shows optional combinations of two nucleotide sequences.
FIG. 5 is a view which shows proportion of the gene detected by the gene expression profile according to the embodiment of the present invention.
FIG. 6 is a view which shows preferable examples of an "X" adaptor and a "Y" adaptor.
FIG. 7 is a view showing the gene sequence of a portion of human arylamine N-acetyl transferase, obtained from a database.
FIG. 8 is a view which shows one example of information on a fragment obtained by the incision with a restriction enzyme.
FIG. 9 is a view showing a portion of one example of gene expression profile which represents the expression of p21.
FIG. 10 is a view showing a portion of one example of gene expression profile which represents the expression of mdm2.
FIG. 11 is a view showing a portion of one example of gene expression profile which represents the expression of cyclinG.
FIG. 12 is a view which shows the compositions of a set of mRNA preparations used in example 2.
FIG. 13 is view which shows a portion of the gene expression profile obtained in example 2.
FIG. 14 is a view which shows a portion of the gene expression profile obtained in example 2.

### Best Mode for Carrying Out the Invention

### 1. Summary of the Invention

The inventors of the present invention have discovered that the degree of complexity of an operation related to the production of a gene expression profile, as well as the cost performance, significantly varies depending on the manner in which a gene expressed in a specific cell is classified. As a result of careful study on the basis of this discovery, the inventors have achieved the present invention.

According to one embodiment of the present invention, a method is provided which enables producing, at a time and in a simple and easy manner, a gene expression profile covering such a wide range as including substantially all of the genes expressed in a specific cell. As substantially all of the expressed genes can be identified, a remarkably large number of expressed genes can be identified, as compared with the conventional method.

Specifically, the one embodiment of the present invention relates to a gene expression profiling method, which has been developed on the basis of the length of the DNA fragment cut with a restriction enzyme and an application of the polymerase chain reaction (i.e., PCR). By using such a gene expression profiling method, almost all of the expressed genes, in other words, both the known and unknown genes, can similarly be identified. Further, this method enables detecting all of the respective genes, without fail, while identifying each of the genes. Further, it is possible to determine the expression of the respective genes.

One essential aspect of the method of producing gene expression profile according to the present invention lies in classifying the genes expressed in a specific cell, as described below. It is assumed that approximately 20,000 types of mRNA are expressed in a specific cell. First, cDNA is synthesized from each of the expressed mRNA preparations. The obtained double-strand cDNA is cut with two appropriate types of restriction enzymes, whereby a fragment of the cDNA having identifiable length is produced for each of the expressed genes. Thereafter, the genes are classified into 256 fractions by identifying the sequence at a portion of the fragments thereof obtained as described above. This classification process is carried out by using the 256 types of primer sets which have been designed in advance. While the relative amount or magnitude of expression is still being reflected therein, the aforementioned fragments are amplified for each primer set or several primer sets, and then the fragments are classified. Each of the fractions, e.g., 256 fractions obtained as a result of classification, is subjected to electrophoresis, and the components of each fraction are separated. In this way, the information of the expressed genes obtained from a cell is subjected to classification to the analyzable level. As a result, a gene expression profile which enables accurately grasping, without fail, the magnitude of expression of each gene for substantially all of the expressed genes, can be produced in a simple and easy manner.

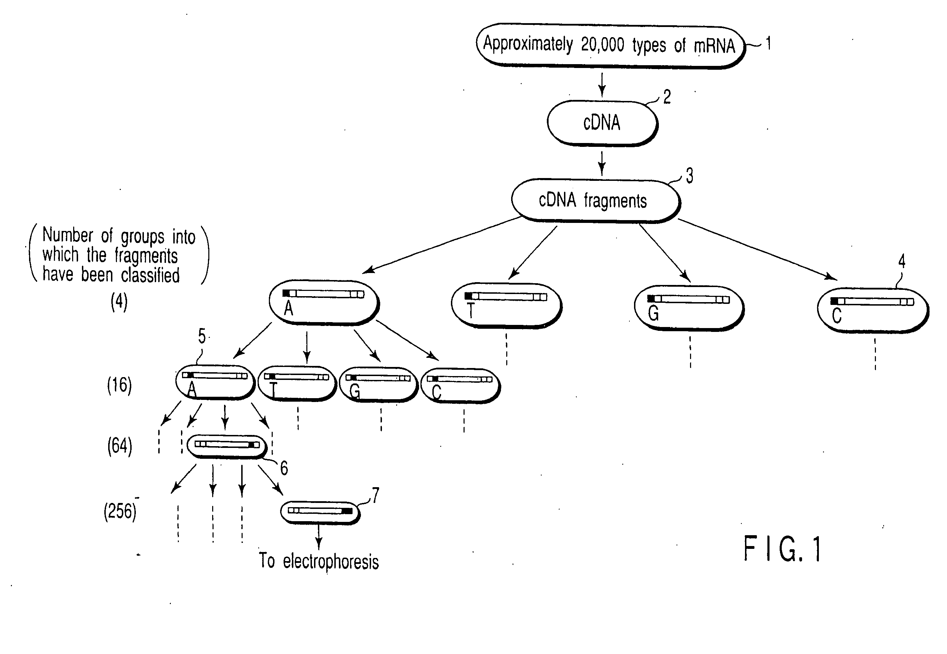

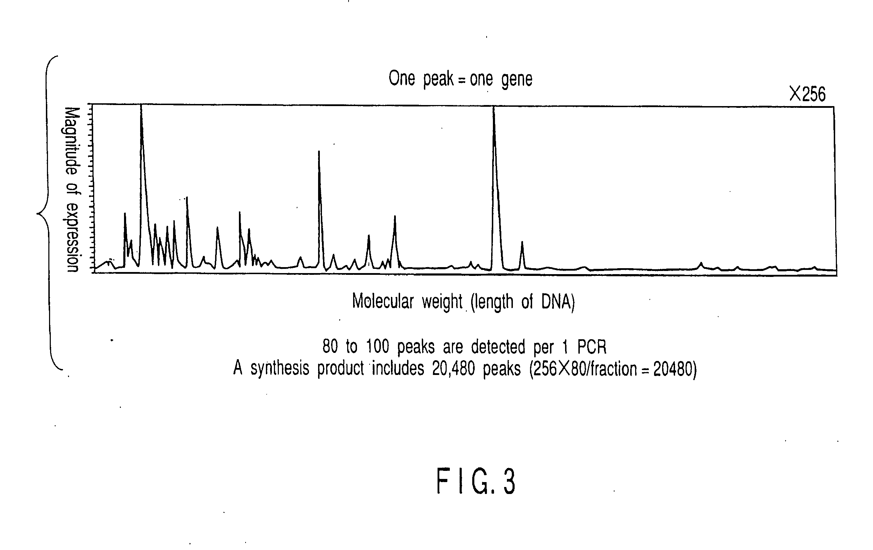

A specific means for classification, e.g., classification into 256 fractions, is described by using FIG. 1. The cDNA group 2 is synthesized from the group 1 consisting of the expressed mRNA preparations. Each of the cDNA is cut with two appropriate types of restriction enzymes, and thereby the cDNA fragment group 3 is obtained. Each cDNA fragment is classified according to the sequence of the two bases at each end (i.e., totally four bases) thereof. In other words, each cDNA fragment is classified according to the type of the two bases at each end thereof, the type including adenine (which will be referred to as "A" hereinafter), guanine (which will be referred to as "G" hereinafter), cytosine (which will be referred to as "C" hereinafter) and thymine (which will be referred to as "T" hereinafter). Specifically, the cDNA fragments are first classified into four groups 4 according to the type of the base at the 5' terminal (which base is shown in black in FIG. 1), then classified into sixteen groups 5 according to the type of the next base, then into sixty-four groups 6 according to the type of the second base at the 3' terminal, and further into 256 groups 7 according to the type of the first base at the 3' terminal. Based on the types of mRNA which are generally expressed, approximately 80 to 100 types of cDNA are assumed to be included in each of the 256 groups 7 which are obtained eventually. That is, when the cDNA of each group is subjected to electrophoresis, it is assumed that approximately 80 to 100 peaks will be detected. Accordingly, all of the mRNA obtained from a specific cell are expressed in 256 types of chart each exhibiting approximately 80 to 100 peaks. These 256 types of chart constitute a profile of the expressed gene. FIG. 3 shows one example of a chart contained in such a profile. The chart of FIG. 3 is a chart showing the components contained in a fraction, which fraction has been obtained as a result of the fragment-classification, subsequent PCR amplification and electrophoresis of the reaction product of each fraction.

Another aspect of the present invention lies in appropriate cutting of cDNA obtained from the expressed mRNA with two appropriate types of restriction enzymes, which are preferably MspI and MseI. Such appropriate cutting result in successfully carrying out the above-mentioned classification. The present invention will be described in more detain hereinafter.

### 2. Detailed Description of the Embodiments

### (1) Gene expression profile

The method of producing a gene expression profile of the present invention basically includes:
(a) a step of synthesizing cDNA from mRNA extracted from a cell, such that a tag substance is added to the 5' terminal of the cDNA;
(b) a step of cutting the product obtained as a result of the reaction in step (a) with a first restriction enzyme X;
(c) a step of connecting, to a fragment obtained in step (b), an "X" adaptor having a sequence complementary to a sequence of a site of the fragment at which site the incision with the first restriction enzyme X has been effected;
(d) a step of connecting the fragment obtained in step (c) to a substance having high affinity with respect to the tag substance, thereby collecting the fragment;
(e) a step of cutting the fragment collected in step (d) with a second restriction enzyme Y and removing a fragment connected to the tag substance, thereby obtaining a fragment including the 5' side-portion of the cut cDNA;
(f) a step of adding, to a fragment obtained in step (e), a "Y" adaptor having a sequence complementary to a sequence of a site of the fragment at which site the incision with the second restriction enzyme Y has been effected;
(g) a step of carrying out a PCR reaction, for the fragment obtained in step (f), by using a primer which has a sequence complementary to the sequence of the "X" adaptor and has two-nucleotide sequence (NN) at the 3' terminal thereof, and a primer which has a sequence complementary to the sequence of the "Y" adaptor and has two-nucleotide sequence (NN) at the 3' terminal thereof; and
(h) a step of subjecting the obtained PCR product to electrophoresis and detecting a migration distance and a peak, thereby producing a gene expression profile.

In the present specification, "the 5' side of double strand DNA" generally represents the 5' side of a sense strand (a sequence homologous with the mRNA as a template) and "the 3' side of double strand DNA" generally represents the 3' side of such a sense strand.

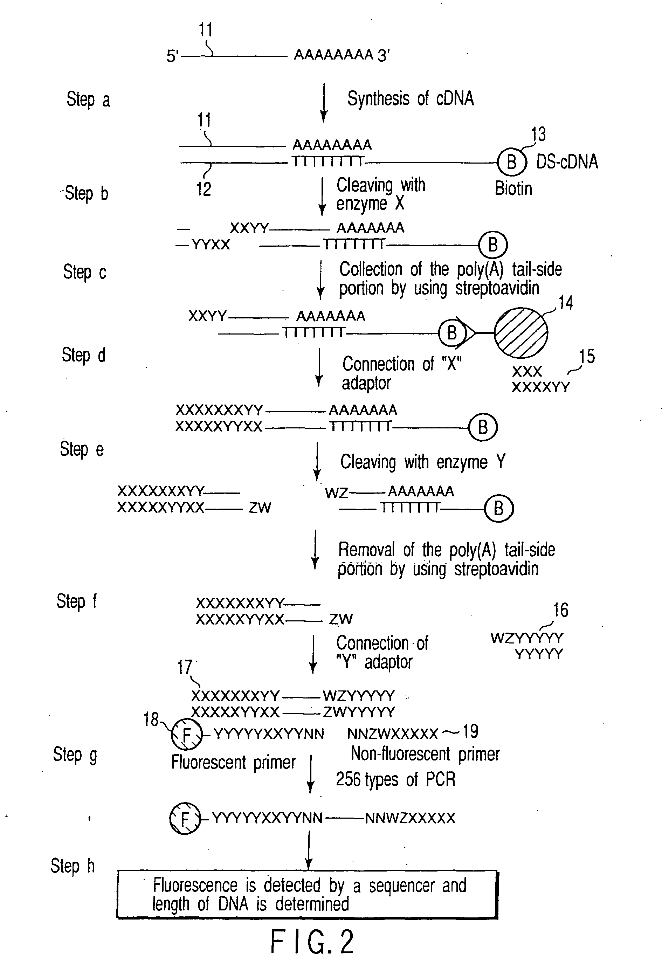

A specific example of the method of producing gene expression profile according to the present invention is described heinafter with reference to FIG. 2. In FIG. 2, each alphabet letter represents a base which constitutes a nucleotide sequence. "A" represents adenine (in other words, adenine will be referred to as "A" hereinafter), "G" represents guanine (in other words, guanine will be referred to as "G" hereinafter), "C" represents cytosine (in other words, cytosine will be referred to as "C" hereinafter) and "T" represents thymine (in other words, thymine will be referred to as "T" hereinafter). Further, "N", "W", "X", "Y" and "Z" each represents any suitable or optional base. X and Y complementarily bind to each other, and W and Z complementarily bind to each other. Note that aforementioned steps (a) to (h) each correspond to steps (a) to (h) of FIG. 2, respectively, exactly in the alphabetical order.

First, mRNA 11 is extracted from a specific cell as the test subject.

An oligo dt primer, which is complementary to the poly(A) tail at the 3' terminal of the mRNA 11 extracted as described above, is marked with biotin 13. A cDNA is synthesized by using the marked mRNA as a primer, and thereby a double strand 12 is obtained (FIG. 2, step (a)). Here, an example in which biotin is used as the tag substance is shown.

The double strand 12 is cut by using MspI, which is a four-base-identifying restriction enzyme, as a first restriction enzyme X (FIG. 2, step (b)). Here, an example in which MspI is used as the first restriction enzyme is shown.

Thereafter, the biotin 13 is captured by using streptoavidin 14. As a result, the 3'-side portion of the cut double strand cDNA is captured (FIG. 2, step (c)). Here, an example, in which streptoavidin is used as a substance having high affinity with respect to the tag substance, is shown.

To the 5' side of the double strand cDNA collected in step (c), an "X" adaptor 15 having a sequence complementary to the identification-incision site of the cDNA at which site the incision with the first restriction enzyme X i.e., MspI, has been effected, is connected (FIG. 2, step (d)).

The resulting product is cut by using a restriction enzyme MseI as a second restriction enzyme Y (FIG. 2, step (e)). Here, an example in which MseI is used as the second restriction enzyme is shown.

Next, a "Y" adaptor 16 having a sequence complementary to the identification-incision site of the cDNA at which site incision with the second restriction enzyme Y i.e., MseI, has been effected, is added or connected (FIG. 2, step (f)). As a result of the above-mentioned treatments, double strand sequence 17 including known sequences at both ends thereof is constructed.

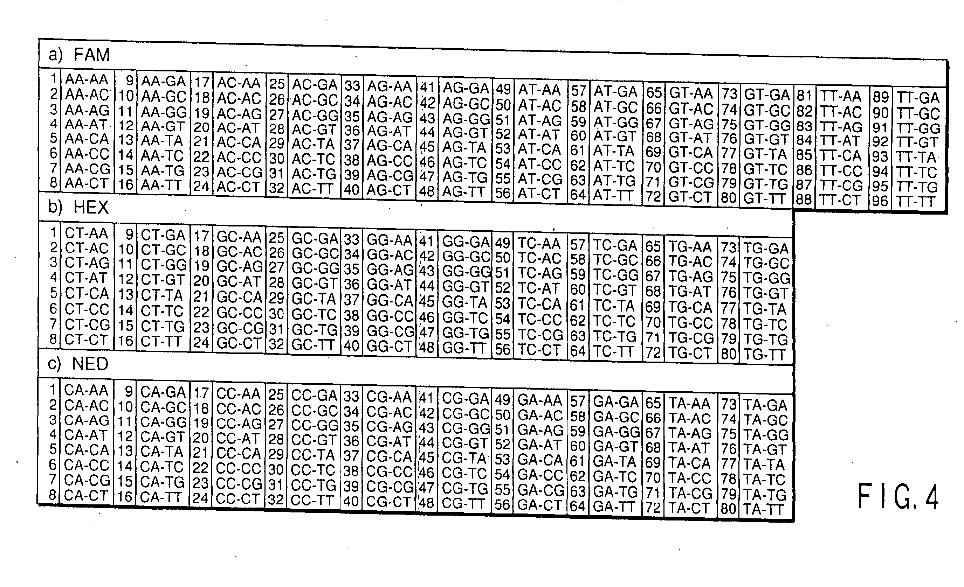

Next, a PCR reaction is carried out by using the double strand sequence 17 as a template, and using a PCR primer 18 at the 5' side of the double strand cDNA marked with a fluorescent colorant (for the antisense strand) and a primer 19 at the 3' side of the double strand cDNA without fluorescent marking (for the sense strand) (FIG. 2, step (g)). Here, the "X" primer 18 and the "Y" primer 19 for the PCR have and utilize sequences which are complementary to the sequences of X adaptor and Y adaptor another sequence including two bases located next to the one sequence in the direction of amplification thereof. As each pair of two bases at the 5' side/the 3' side (i.e., the totally four bases derived from both terminals) is designed such that each of the four bases can be any of the four types of bases A, G, C and T, totally 256 types of primer set can be obtained. Accordingly, by carrying out PCR for all of the thus prepared double strand cDNA preparations, it is possible to classify all of the existing cDNA preparations into 256 groups and carrying out PCR amplification therefor without fluorescent marking. FIG. 4 shows the combinations of the four bases, in which the sequence of the four bases are optionally decided, of the primer set. FIG. 4 discloses the combinations ranging from AA-AA to TA-GA.

In step (h) of FIG. 2 as the final process, the PCR products obtained as 256 types of fractions are subjected to electrophoresis and peaks of each case or fraction are measured, whereby a gene expression profile is obtained (FIG. 2, step (h)). FIG. 3 shows a chart which is an example of the result obtained by subjecting one of the 256 fractions prepared as described above to electrophoresis. In FIG. 3, the Y-axis of the graph indicates the magnitude of expression, with fluorescent strength being used as the index, and the X-axis of the graph indicates the molecular weight, with the migration distance at electrophoresis being used as the index.

It is acceptable to exchange step (c) and step (d) in the order. That is, step (d) may be carried out prior to step (c).

Further, one restriction enzyme which is used as the first restriction enzyme may be used as the second restriction enzyme, while another restriction enzyme which is used as the second restriction enzyme is used as the first restriction enzyme. As a result, incision is made possible for a larger number of genes and thereby the detection sensitivity is enhanced.

Specifically, the double strand 12 obtained in step (a) is divided into two groups i.e., cDNA mix A and cDNA mix B. It is acceptable that the cDNA mix A is subjected to the treatment of steps (b) to (h) as described above, and simultaneous with or after the treatment of the cDNA mix A, the cDNA mix B is subjected to the following treatment. Specifically, the cDNA mix B is treated in a manner similar to that of the above-mentioned method, except that the restriction enzyme MseI is used as the first restriction enzyme and the restriction enzyme MspI is used as the second restriction enzyme. By using the first restriction enzyme and the second restriction enzyme in the exchanged manner, the genes which would not be detected had the restriction enzymes not been exchanged can also be detected.

More specifically, in the treatment of the cDNA mix B, the double strand 12 contained in the cDNA mix B is cut with MseI, which is a four-base-identifying restriction enzyme. Thereafter, to the identification-incision site of the cDNA at which site the incision with the restriction enzyme MseI has been effected, the MseI adaptor having a sequence complementary to the identification-incision site is connected or bound. Then, biotin is captured by using streptoavidin, and thereby the 3'-side portion of the cut double strand 12 is collected. Next, the collected 3'-side portion of the double strand 12 is cut with the restriction enzyme MspI. Thereafter, to the identification-incision site of the cDNA at which site the incision with the restriction enzyme MspI has been effected, the MspI adaptor having a sequence complementary to the identification-incision site is bound. As a result of the above-mentioned treatment, a sequence including the double strand 12 with known sequences connected to both terminals thereof is constructed. Next, for the obtained sequence, a PCR reaction for cDNA is carried out by using an X primer 18 marked with a fluorescent colorant and a Y primer 19 without fluorescent marking. Here, the primer 18 and the primer 19, having sequences complementary to the X adaptor and the Y adaptor another sequences of two bases located next to the sequences in the direction of amplification thereof, is used. As each pair of two bases at the 5' side/the 3' side (i.e., the totally four bases derived from both terminals) is designed so that each of the four bases can be any of the four types of bases A, G, C and T, totally 256 types (combinations) of primer,set can be obtained (Refer to the steps (a) to (f) of FIG. 2. The 256 types of the NN-NN nucleotide sequence are specifically shown in FIG. 4). Accordingly, by carrying out PCR for all of the cDNA preparations by using these primer sets, it is possible to classify all the existing types of cDNA preparations into 256 groups. The PCR products obtained as 256 types of fractions are subjected to electrophoresis and migration distance and peaks of each case or fraction are measured, whereby a gene expression profile is obtained.

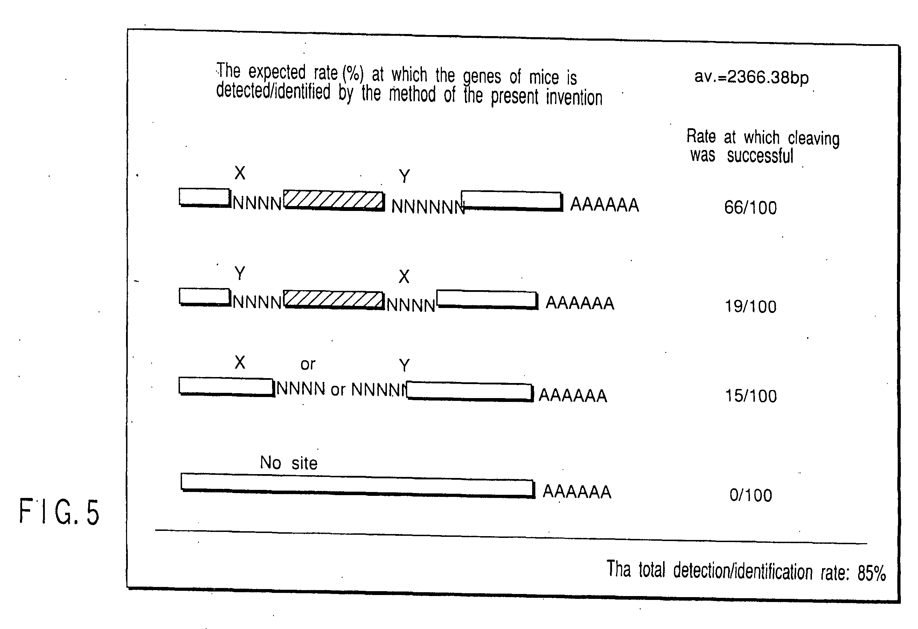

Regarding the expressed genes which are classified according to the method of the present invention, in the case of mouse, for example, approximately 85% of 100 genes of mouse selected at random can be identified and detected, as shown in FIG. 5. Specifically, when MspI is used as the first restriction enzyme and MseI is used as the second restriction enzyme, approximately 66% of the expressed genes goes through incision. When MseI is used as the first restriction enzyme and MspI is used as the second restriction enzyme, approximately 19% of the expressed genes goes through incision.
Accordingly, by exchanging the first restriction enzyme and the second restriction enzyme in the order in use thereof, approximately 85% of the expressed genes can be identified and detected, as a whole. Due to this, a gene profile can be produced more accurately than in the conventional method. The proportion of genes which can be identified by the conventional method is generally 20 to 30%, and 50% at most. Therefore, the proportion of genes which can be identified by the gene expression profile produced by the method of the present invention is remarkably higher than the proportion achieved by the conventional method. It is concluded that the method of the present invention enables identifying substantially all of the genes contained in a cell.

The term "gene expression profile" used in the present specification represents information including an expression pattern of genes in a specific cell in a given condition, absence/presence of expression of known and unknown genes, the magnitude of expression of all the expressed genes, and the like. The gene expression profile produced by the method of the present invention can be used as a means for analyzing expression of genes.

The term "poly(A) tail" used in the present specification represents a sequence at the 3' terminal of mRNA, which is, in general, also referred to as "poly(A)". cDNA can be synthesized from mRNA having the aforementioned poly(A) tail by using the "oligo dT primer" having a sequence complementary to the poly(A) tail. The "oligo dT primer" used in the present invention is, in general, also, referred to as "oligo(dT) primer". The synthesis of cDNA by using the oligo dT primer can be achieved in any suitable conditions which are generally applied to the conventional method.

The "tag substance" and the "substance having high affinity with respect to the tag substance" used in the present invention are substances which can specifically bind to each another with high affinity, thereby forming a binding pair. Although biotin is used as the tag substance and streptoavidin is used as the substance having high affinity with respect to the tag substance in the example described in the aforementioned item "(1) Gene expression profile", the types of the tag substance and the substance having high affinity with respect to the tag substance are not limited to these specific examples. Any binding pair can be used as long as the pair exhibits specific binding with high affinity therebetween. Examples of the combination of the tag substance and the substance having high affinity with respect to the tag substance, which can be employed in the present invention, include: biotin and streptoavidin; biotin and avidin; FITC and FITC antibody; DIG and anti-DIG; protein A and mouse IgG; latex particles; and the like. However, the types of the tag substance and the substance having high affinity with respect to the tag substance are not limited to the aforementioned examples. Further, in each of the combinations described above, each of the two substances can be used as either the tag substance or the substance having high affinity with respect to the tag substance.

The "restriction enzyme" used in the present invention is an enzyme which is, in general, also referred to as "restriction endonuclease" and effects hydrolysis and incision of double strand DNA at a specific sequence. In the method according to the present invention, two types of restriction enzymes X and Y are used in combination, in order to obtain appropriate fragments. As the restriction enzyme which can be used in the present invention, an enzyme capable of cutting the double strand, constituted of cDNA which has been synthesized from mRNA as the expressed gene, to a fragment having identifiable length, is preferable. It is preferable that the enzyme is capable of cutting as many of the obtained double strands as possible, and it is more preferable that the enzyme is capable of cutting substantially all of the obtained double strands. Table 1 shows examples of such enzymes. It is acceptable to select any two enzymes from Table 1 and use these enzymes in combination. All of the enzymes shown in Table 1 are four-base-identifying enzymes. Alternatively, four-base-identifying enzymes of the types other than those of Table 1 or six-base-identifying enzymes may be used. In the method according to the present invention, it is preferable that four-base-identifying enzymes are used, and it is more preferable that MspI and MseI are used in combination. In the aforementioned example, MspI (or MseI) is used as the restriction enzyme X, and MseI (or MspI) is used as the restriction enzyme Y.

**Table 1**

| | | | | |
|---|---|---|---|---|
| AccII | CG/CG | | HpaII | C/CGG |
| AlaI | GT/AC | | Hsp92II | CATG/ |
| AluI | AG/CT | | HspAI | G/CGC |
| AspLEI | GCG/C | | Kzo9I | /GATC |
| BfaI | C/TAG | | MaeI | C/TAG |
| BscFI | /GATC | | MboI | /GATC |
| Bsh1236I | CG/CG | | MseI | T/TAA |
| BshI | GG/CC | | MspI | C/CGG |
| BsiSI | C/CGG | | MvnI | CG/CG |
| Bsp143I | /GATC | | NdeII | /GATC |
| BstUI | CG/CG | | NlaIII | CATG/ |
| BsuRI | GG/CC | | PalI | GG/CC |
| CfoI | GCG/C | | RsaI | GT/AC |
| Csp6I | G/TAC | | Sau3AI | /GATC |
| DpnII | /GATC | | Sse9I | /AATT |
| FnuDII | CG/CG | | TaqI | T/CGA |
| HaeIII | GG/CC | | ThaI | CG/CG |
| HapII | C/CGG | | TrulI | T/TAA |
| HhaI | GCG/C | | Tru9I | T/TAA |
| Hin2I | C/CGG | | Tsp509I | /AATT |
| Hin6I | G/CGC | | TspEI | /AATT |
| HinP1I | G/CGC | | TthHB8I | T/CGA |

The "adaptor" employed in the present invention is used for effecting connection of the primers which work in the final PCR amplification. The adaptor used in the present invention is designed in accordance with the restriction enzymes to be used. Specifically, the "X" adaptor to be connected to the identification-incision site at which the incision with the restriction enzyme X has been effected may include a sequence complementary to the identification-incision site (at which the incision with the restriction enzyme X has been effected) and another optional sequence. The type of another optional sequence and the base-length thereof can be designed in consideration of the factors such as the efficiency of PCR. It is preferable that the "X" adaptor is designed such that the "X" adaptor has approximately 15 bases. Such a structure of the "X" adaptor results in the stable performance of PCR. The "Y" adaptor to be connected to the identification-incision site at which the incision with the restriction enzyme Y has been effected may include a sequence complementary to the identification-incision site (at which the incision with the restriction enzyme Y has been effected) and another optional sequence. The type of another optional sequence and the base-length thereof can be designed in consideration of the factors such as the efficiency of PCR. It is preferable that the "Y" adaptor is designed such that the "Y" adaptor has approximately 15 bases. Such, a structure of the "Y" adaptor results in the stable performance of PCR.

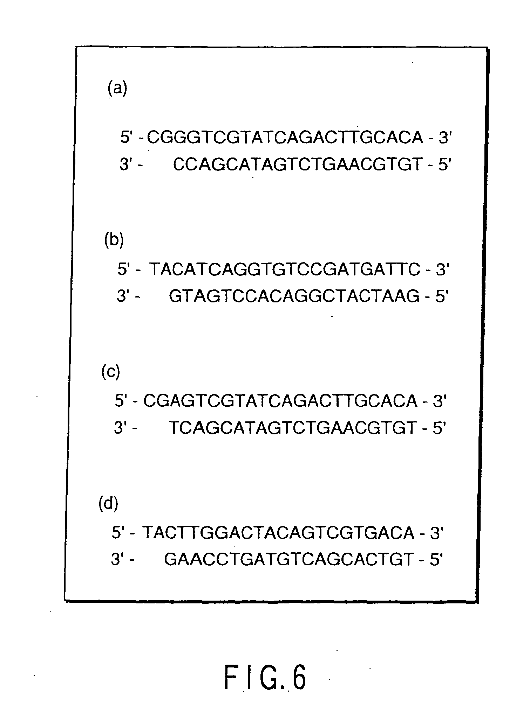

A preferable example of the sequence of the "X" adaptor in the case in which MspI is used as the restriction enzyme X is shown in FIG. 6(a). A preferable example of the sequence of the "X" adaptor in the case in which MseI is used as the restriction enzyme X is shown in FIG. 6(b). Further, a preferable example of the sequence of the "Y" adaptor in the case in which MspI is used as the restriction enzyme Y is shown in FIG. 6(c), and a preferable example of the sequence of the "Y" adaptor in the case in which MseI is used as the restriction enzyme Y is shown in FIG. 6(d). However, the sequence of the "X" adaptor and that of the "Y" adaptor are not limited to the examples shown in FIGS. 6(a) to 6(d).

The "primer set" used in step (g) includes a pair of primers, primer "X" and primer "Y", which primers are used for amplifying by PCR the double strand cDNA obtained in step (f). The details of the primer set are as described above. The "two nucleotide-sequence (NN)" used in the present invention is a sequence optionally selected from adenine, thymine, guanine and cytosine. As described above, in a case in which the optional bases are constituted of two bases (i.e., NN), a chart obtained as result of PCR of one sample includes approximately 80 to 100 peaks. In the method of the present invention, each "optional sequence" at each side is designed as a two-nucleotide sequence, in consideration of the convenience in operation and precision in analysis in the method. Accordingly, in the method according to the present invention, the "optional sequence" at each side is preferably a two-nucleotide sequence (NN) and the number of the primer set is preferably 256. However, the type of the "optional sequence" and the number of the primer set are not limited to the above-mentioned examples. It is acceptable that the two-nucleotide sequence NN of at least one of the two primers (i.e., the "X" primer and/or the "Y" primer) is replaced with a sequence including no less than three bases. When the number of the bases included in the "optional sequence" is increased, the number of types of primers included in the primer set is also increased. When the two-nucleotide sequence NN of one of the two primers is replaced with a three-nucleotide sequence, 1024 or 4096 fractions will be obtained.

Further, in the present invention, it is preferable that a fluorescent material is bound to one terminal of one of the primers of each primer set so that the detection thereof after PCR can be facilitated. Specifically, it is preferable that a fluorescent material is bound to the 5' terminal of the "X" primer having a sequence complementary to the "X" adaptor. Examples of the fluorescent material which can be used in the method of the present invention include 6-carboxyfluorescein (which will be referred to as "FAM" hereinafter), 4,7,2',4',5',7'-hexachloro-6-carboxyfluorescein (which will be referred to as "HEX" hereinafter), NED (manufactured by Applied Biosystems Japan Co., Ltd.), 6-carboxy-X-rhodamine (which will be referred to as "Rox" hereinafter) and the like.

The PCR reaction carried out according to the invention may be carried out in a condition generally applied to the conventional method. For example, the PCR reaction can be carried out in the condition of 95°C for 1 minute, (95°C for 20 seconds, 68°C for 30 seconds, 72°C for 1 minute) × 28 times, and 60°C for 30 minutes.

The means for conducting electrophoresis which can be used in the present invention may be any means for electrophoresis, in general, as long as the means enables separation of reagents according to the molecular weight thereof. Commonly used devices for electrophoresis can be used, whose examples include a sequencer, ABI PRISM 3100 (manufactured by Applied Biosystems Japan Co., Ltd.), ABI PRISM 3700 (manufactured by Applied Biosystems Japan Co., Ltd.), and MegaBACE 1000 (manufactured by Amersham Pharmacia Co., Ltd).

### (3) Identification of peaks

Further, according to the present invention, it is possible to identify the gene represented by each peak of the chart obtained as described above. Due to identifying the peak, it is possible to identify the gene(s) which is/are expressed or whose expression magnitude is increased/decreased in a specific environment.

Identification of the gene can be carried out by collecting the molecule or gene exhibiting a particular peak in the chart, and determining the sequence thereof by a laboratory operation including the common method such as sequencing.

Alternatively, it is possible to theoretically identify the gene by using a computer, without relying on a laboratory operation as described above. For example, it is possible to identify the gene by using a computer, on the basis of data of the identification site of the restriction enzyme in use, data of the molecular weight of the fragment obtained by the incision with the restriction enzyme, and data which is available from the free database.

The length of the fragment, observed when a gene sequence optionally selected from the database is cut with a specific restriction enzyme, as well as the details of the identification site of the restriction enzyme, can easily be determined on a display of a computer. On the other hand, the length of the fragment, observed after the incision with the restriction enzymes used in the method of the present invention, is clearly known from the result of electrophoresis. Accordingly, by further considering the adaptor sequence in use, it is possible to determine from which gene the fragment is derived, without necessitating any laborious analysis by experients in a laboratory. One example of the method conducting such theoretical identification by using a computer will be described in example 1 below.

A computer for common use can be used in the present invention. For example, a computer device equipped with an input section including a keyboard, a mouse and the like, an output section including a printer, a display and the like, and a computing section such as CPU, can be used.

Examples of the database from which useful data can be obtained include public data banks such as GenBank, EMBL and DDBJ, commercial databases and the like, with no restriction to these examples.

Further, it is also possible to combine the method relying on a laboratory operation and the method based on theoretical computation by a computer, in the aforementioned gene identification process.

### (4) Analysis on gene expression

In the method of producing gene expression profile according to the present invention, the magnitude of expression of each gene expressed in the subject cell is reflected on the magnitude of the peak corresponding to the gene shown in the chart. Accordingly, by observing the change in the magnitude of the peaks, the expression of each gene can be analyzed.

For example, it is possible to make comparison, with regards to the expression of a gene, between a normal cell and an abnormal cell, between a normal cell and a cancer cell, between cells different in type, and between cells treated in different conditions.

Further, if a gene which expresses itself or whose expression magnitude is changed as result of a specific stimulus is identified by the method of the present invention in advance, it suffices, in the tests thereafter, to use only the primers corresponding to the specific gene and produce the gene expression profile resulted from the primer. The expression of the targeted gene can be analyzed on the basis of the gene expression profile obtained in such a manner.

### Example 1

Influence of radioactive ray irradiation on the magnitude of expression of p21, mdm2 and cyclin G

The influence of radioactive ray irradiation on the magnitude of expression of p21, mdm2 and cyclin G was studied, as described below. A gene expression profile was produced by using mRNA obtained from a mice mammary cancer cell stock SR-1 which had been subjected to radioactive ray irradiation. Another gene expression profile was produced by using mRNA obtained from a mice mammary cancer cell stock SR-1 which had not been subjected to radioactive ray irradiation. The two gene expression profiles were compared with each other.

### 1. Production of gene expression profiles

The gene expression profiles were actually produced according to the method of the present invention.

### 1-1. Extraction of mRNA and synthesis of cDNA

Mice mammary cancer cell stock SR-1 (donated by Professor Koyama, Yokohama City University) was cultured in an αMEM culture medium set in a 75 cm³ flask (manufactured by Falcon Co., Ltd.). Radioactive rays of 7 Gy were irradiated on the cells, from above, by using a "Pantac", manufactured by Shimadzu Corporation, Ltd. The irradiation time was 3 hours. Mice mammary cancer cell stock SR-1 which had not been subjected to such irradiation was also prepared as a control at the same time. 20 µg of mRNA as the whole weight was extracted from each cell by using a FastTrack 2.0 kit (manufactured by Invitrogen Co., Ltd.).

Each mRNA (20 µg) extracted as described above was mixed with 5'-biotinated oligo dT primer (100 pmole/0.8 µL) (manufactured by BRL Co., Ltd.), and the mixture was incubated at 65°C for 5 minutes. The mixture was then cooled with ice. Thereafter, the mixture was incubated with MgCl₂ (the final concentration thereof was 5 mM), 0.5 mM of dNTP Mix (manufactured by BRL Co., Ltd.) and 10 mM of DTT (manufactured by BRL Co., Ltd.), in 20.0 µL of a reverse transcription buffer, at 42°C for 60 minutes. The resulting product was then incubated with dNTP Mix (manufactured by BRL Co., Ltd., the final concentration thereof was 0.27 mM), 1.33 mM of DTT (manufactured by BRL Co., Ltd.), 20.0 units of E. coli ligase (manufactured by BRL Co., Ltd.), 40.0 units of E. coli DNA polymerase (manufactured by BRL Co., Ltd.) and 2.0 units of RNaseH (manufactured by BRL Co., Ltd.), in 150.0 µL of a double strand synthesizing buffer, at first at 16°C for 120 minutes and then 70°C for 15 minutes. Then, the reaction was stopped. The obtained reaction product was equally divided into two portions (the reaction product mixture A and the reaction product mixture B).

### 1-2. Treatment of the reaction product mixture A

The reaction product mixture A was treated, as described below. In the present example, MspI was used as the first restriction enzyme and MseI was used as the second restriction enzyme.

First, the restriction enzyme MspI (manufactured by Takara Co., Ltd., the final concentration thereof being 20 units in 100 µL) was reacted with the reaction product mixture A containing 10 µg of mRNA, at 37°C for 360 minutes. After the reaction, the product was purified with ethanol (500 µL × 3 times). Thereafter, the product was subjected to ligation with 5.0 µg of the "X" adaptor having a sequence of GC (i.e., a sequence complementary to the incision fragment site at which site the incision with the restriction enzyme MspI had been effected) (manufactured by BRL Co., Ltd.) and 10 units of T4 DNA ligase (manufactured by NEB Co., Ltd.), in 15 µL of the T4 DNA ligase buffer. Then, magnetic beads having streptoavidin (manufactured by Dinal Co., Ltd.) fixed thereto were added to the reaction solution. The biotin included in the double strand in the reaction solution was bound to streptoavidin fixed to the magnetic beads, whereby a ligation product was obtained.

Next, the ligation product was reacted with the restriction enzyme MseI (manufactured by NEB Co., Ltd., the final concentration thereof was 50 units in 200 µL), at 37°C for 360 minutes. After the reaction, the supernatant thereof was transferred to another tube and was subjected to purification with ethanol (1000 µL × 3 times). Thereafter, the product was , subjected to ligation with 10 pmole of the "Y" adaptor having a sequence of AT (i.e., a sequence complementary to the incision fragment site at which site the incision with the restriction enzyme MseI had been effected) (manufactured by BRL Co., Ltd.) and 10 units of T4 DNA ligase (manufactured by NEB Co., Ltd.), in 10 µL of the T4 DNA ligase buffer.

Next, PCR was carried out with respect to the ligation product obtained as described above. In the present example, one of the three types of fluorescent colorants FAM, HEX and NED was bound to the 5' side of the "X" primer having a sequence complementary to the "X" adaptor. The "X" primer further includes two-nucleotide sequence (NN), at the 3' side thereof, next to the sequence complementary to the "X" adaptor. On the other hand, the "Y" primer having a sequence complementary to the "Y" adaptor further includes two-nucleotide sequence (NN), at the 3' side thereof. The combinations of each fluorescent colorant and each NN are shown in FIG. 4. In FIG. 4, the combinations are classified according to the substance used for marking. That is, the sequences marked with FAM are shown in row (a), the sequences marked with HEX are shown in the row b), and the sequences marked with NED are shown in row (c). The two-nucleotide sequences are expressed as "(NN) of the X primer"-"(NN) of the Y primer". In the present example, three types of fluorescent probes were used in order to enhance the work efficiency. The method of the present invention can be implemented with a single fluorescent probe being used, in a manner similar to that of the case in which three types of fluorescent probes are used.

Specifically, after the ligation was completed, the reaction solution was diluted to 612 µL with Tris-HCl buffer (which buffer will be referred to as "TE" hereinafter). 1 µL of a solution containing the primer represented by the first sequence of "FAM" row (a) of FIG. 4 (i.e., "AA-AA"), 1 µL of a solution containing the primer represented by the first sequence of "HEX" row (b) of FIG. 4 (i.e., "CT-AA"), and 1 µL of a solution containing the primer represented by the first sequence of "NED" row (c) of FIG. 4 (i.e., "CA-AA"), were mixed together and then the mixture was mixed with 1 µL of the diluted reaction solution. Similarly, a solution mixture of a set of the three primers, represented by the sequences derived from rows (a), (b) and (c) and sharing the same reference number, was prepared and the solution mixture was mixed with 1 µL of the diluted reaction solution, in a manner similar to that described above. As the primers from No. 81 to No. 96 in the FAM row do not have corresponding primers in the HEX and NED rows, the primer solutions of No. 81 to No. 96 in the FAM row were mixed with 1 µL of the diluted reaction solution, without adding primer solutions of HEX and NED. As a result of the aforementioned operation, the PCR reaction products produced from the 256 types of primer sets were converted to 96 samples for electrophoresis.

These 96 samples for electrophoresis were then subjected to electrophoresis. The electrophoresis was carried out under the condition of a migration voltage of 15 kV and a migration time of 2000 seconds, with a capillary sequencer (ABI PRISM 3100, Applied Biosystems Japan Co., Ltd.). The result of the electrophoresis was obtained, for each sample, as a chart in which the X-axis represents the molecular weight shown according to the migration distance, which migration distance being used as an index, and the Y-axis represents the magnitude of gene expression, shown according to the fluorescent intensity, which fluorescent intensity being used as an index. One sample includes the PCR products marked with the three different types of fluorescent materials. However, these PCR products (or the three different types of fluorescent materials) can be identified by changing the wavelength to be applied.

### 1-3. Treatment of the reaction product mixture B

The reaction product mixture B was subjected to a treatment in a manner similar to that in the treatment of the reaction product mixture A, except that the MseI was used as the first restriction enzyme and MspI was used as the second restriction enzyme. Thereafter, 96 samples obtained from the reaction product mixture B were subjected to electrophoresis in a manner similar to that in the reaction product mixture A, to obtain charts.

According to the method similar to that described in the aforementioned 1-1 and 1-2, the gene expression profile was obtained as the charts representing the results of the electrophoresis.

### 1-4. Analysis of the gene database

With regard to the peaks detected in the gene expression profile obtained as described above, information on the incision site at which the incision with the restriction enzyme in use had been effected and information on the fragments produced as a result of the incision were obtained, by using data in the database, in order to identify the genes represented by these peaks.

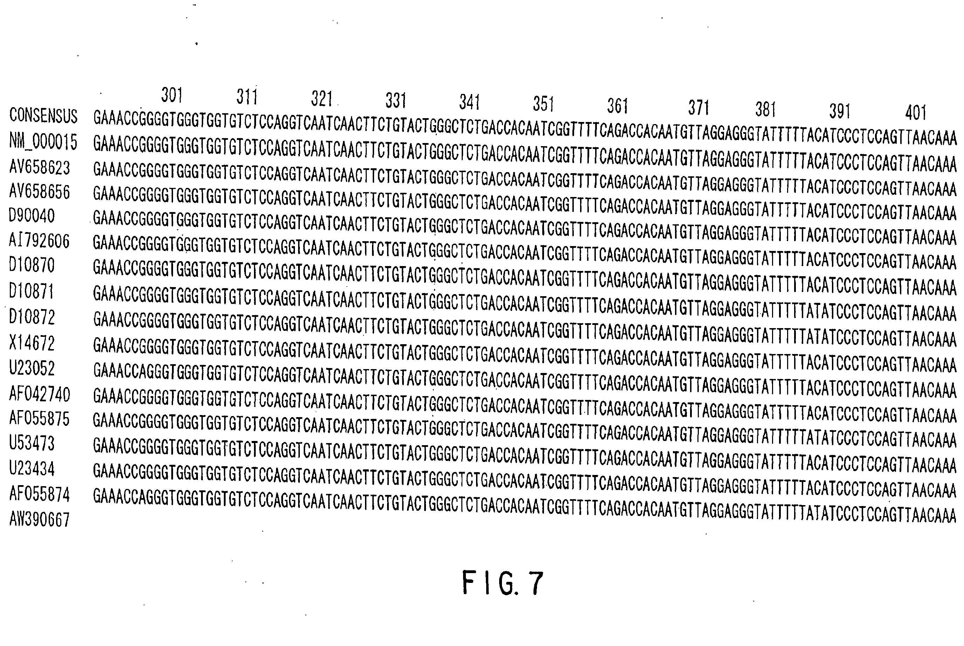

First, the data from the gene database of GenBank, on the genes the length of whose mRNA had been revealed, and the data from EST, on the genes only a portion of whose sequence had been registered, were all accumulated, and the data derived from each gene was classified as one group, according to the type thereof. A consensus sequence was obtained from the accumulated data. FIG. 7 shows the consensus sequence and a portion of the sequence used for arranging the consensus sequence (refer to FIG. 7). The sequence indicated at the top of FIG. 7 is the consensus sequence. The term "consensus sequence" used in the present specification represents a sequence of one type of gene, obtained by determining for each portion of the sequence a base which appears at the highest rate among all of the plural sequences which have been determined with regards to the gene. FIG. 6 shows, as one example, a portion of gene sequence of human arylamine N-acetyl transferase.

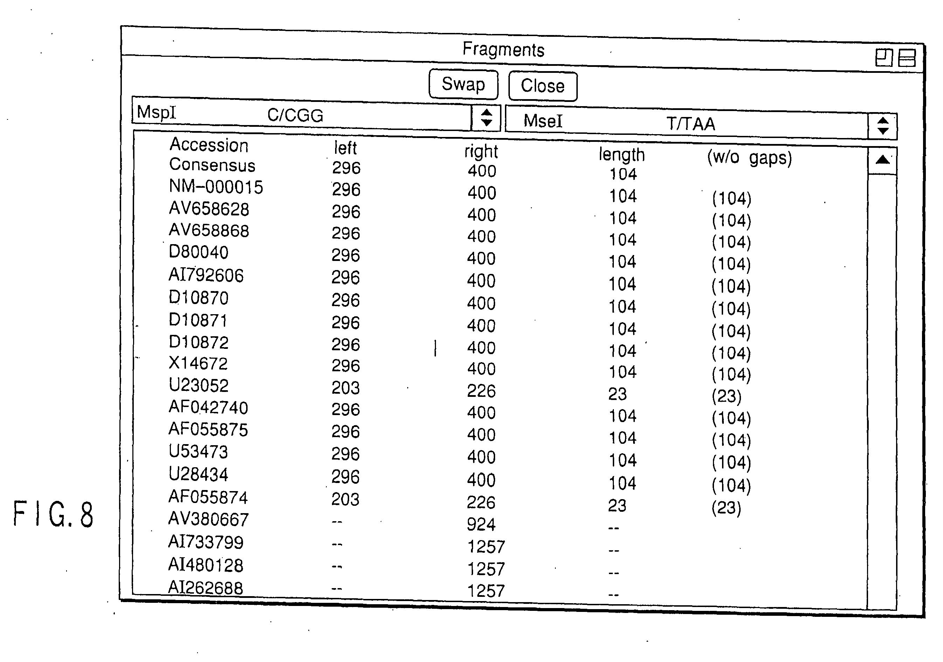

Next, with respect to the consensus sequence and all the data used for determining the consensus sequence, the identification sequence of the restriction enzyme X located closest to the 3' terminal was detected. Thereafter, the identification sequence of the restriction enzyme Y located closest, in the 3' direction, to the identification site of the restriction enzyme X was detected. The identification sequence of MspI is C/CGG, and the incision is effected at the site of "/". The identification sequence of MseI is T/TAA. Further, the number of the bases of DNA, which can be assumed on the basis of the incision fragments of the restriction enzyme X and the restriction enzyme Y obtained as described above, was theoretically calculated. One example of the data obtained as described above is shown in FIG. 8 (refer to FIG. 8).

In FIG. 8, it is understood from the data from the database of GenBank and a number of registered data of EST that an incision fragment of 104 bp is obtained (refer to the "length" column of FIG. 8). However, the data of FIG. 8 also indicates a possibility that some data include mutation or errors in sequence reading, and thereby an incision fragment of 23 bp is also obtained (refer to the "length" column of FIG. 8).

Further, with regards to p21, mdm2, cyclinG and gadd45 among the above-mentioned gene data, which are the genes whose expression is known to be increased as a result of radioactive ray irradiation, the length of the incision fragment and the sequence of the two bases located on the inner side of the restriction enzyme identification site were analyzed.

### 1-5. Identification of genes

By studying the data obtained from the above-mentioned 1-2 and 1-3, together with the data obtained from the above-mentioned 1-4, with comparing the sets of data with each other, the genes represented by the peaks detected at the gene expression profile of the present invention were identified.

On the basis of the length of the incision fragments of p21, mdm2, cyclicG and gadd45 and the sequence of the two bases on the inner side of the restriction enzyme identification site obtained from the databases, it was determined that, from which primer set, i.e., from which set of X primer and Y primer among the 512 types of primers used in the method of the present invention, p21, mdm2, cyclicG and gadd45 were each detected.

Further, the length of DNA, detected in a manner similar to that described above, was revealed. On the basis of the length of DNA, a peak corresponding to the molecular weight matching the revealed DNA length was separated from the peaks representing the molecules separated by the electrophoresis. The nucleotide sequence of the DNA represented by the peak separated as described above was analyzed by sequencing, whereby it was confirmed that the genes were the targeted genes, i.e., p21, mdm2, cyclicG and gadd45.

### 1-6. Influence of radioactive ray irradiation on the expression magnitude of p21, mdm2, cyclicG and gadd45

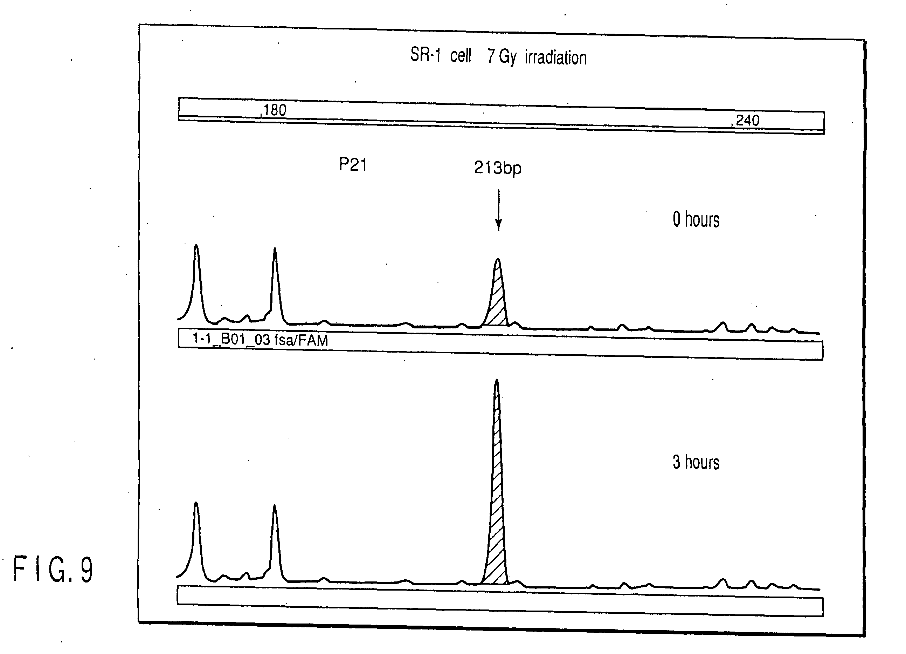

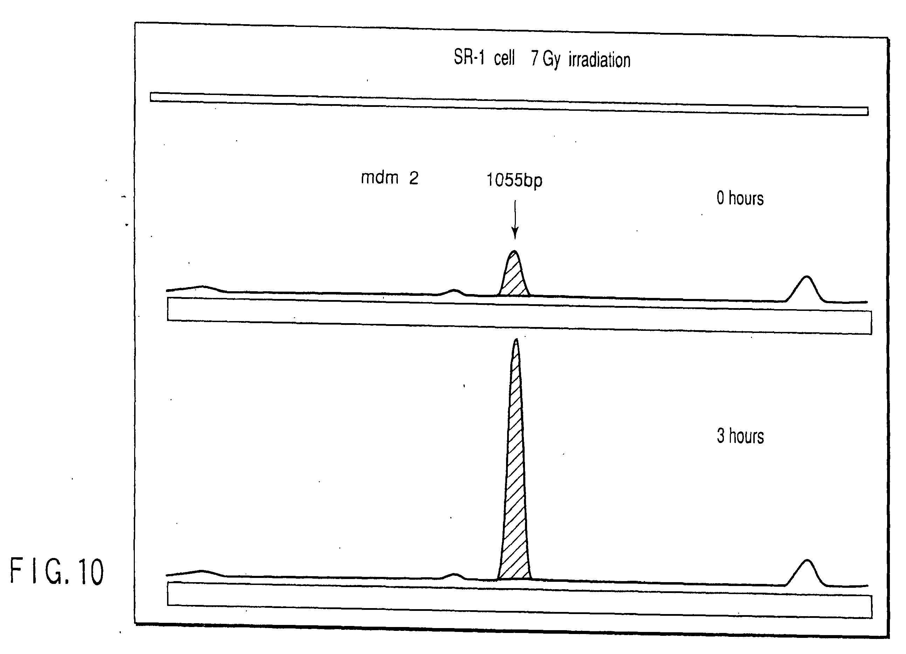

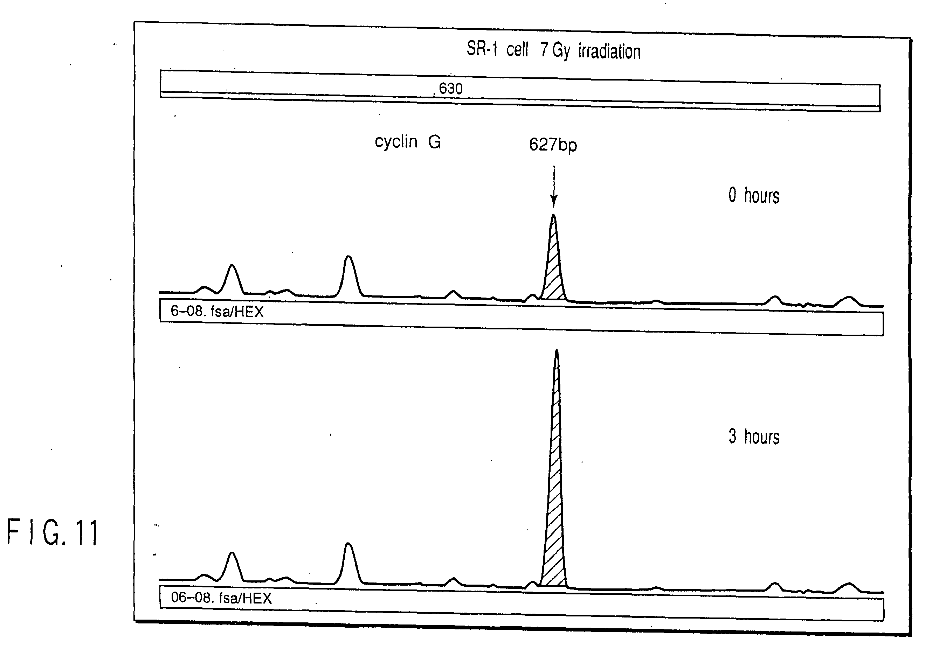

The peaks of the respective genes of p21, mdm2 and cyclicG, obtained according to the method described above, are shown in FIGS. 9, 10 and 11. The upper chart of each of FIGS. 9, 10 and 11 shows a portion of the gene expression profile derived from mRNA obtained from a cell which was not subjected to radioactive ray irradiation. The lower chart of each of FIGS. 9, 10 and 11 shows a portion of the gene expression profile derived from mRNA obtained from a cell which was subjected to 7 Gy radioactive-ray irradiation for 3 hours. The peak of the targeted gene is shown with an arrow.

As shown in FIGS. 9, 10 and 11, it has been confirmed that the expression magnitude is increased by radioactive ray irradiation in each of p21, mdm2 and cyclicG. Further, a similar result was obtained for gadd45, although the data thereof is not shown.

### Example 2

### Analysis of gene expression

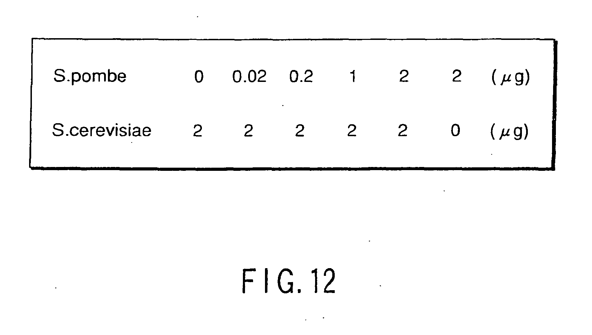

Further, the gene expression was analyzed. Fission yeast (which will be referred to as "S. p." hereinafter) and budding yeast (which will be referred to as "S. c." hereinafter) were used as the cells. For each type of cell, mRNA was extracted in a manner similar to that of example 1. The extracted mRNA of each type of cell was mixed with each other such that the whole amount of mRNA derived from S. p. was varied in a range of 0, 0.02, 0.2, 1, 2 and 2 (µg), while the whole amount of mRNA derived from S. c. was varied in a range of 2, 2, 2, 2, 2 and 0 (µg), as shown in FIG. 12.

For each of the six types of mRNA preparations prepared as described above, a gene expression profile was prepared in a manner similar to that of example 1.

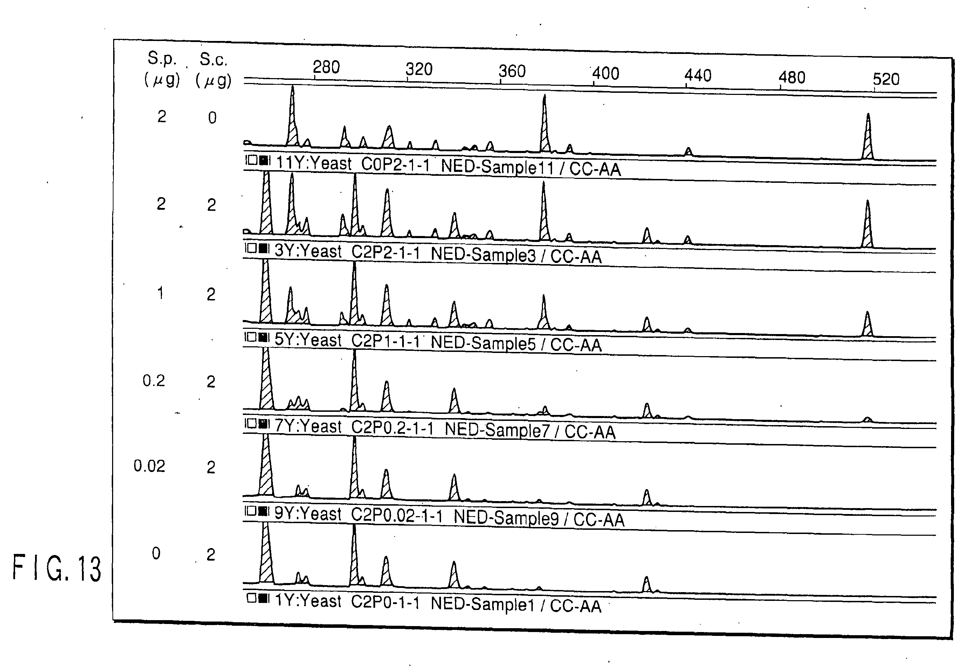

FIG. 13 shows charts representing a portion of the gene expression profile obtained as described above. The composition of the mRNA preparations from which each chart is derived is shown at the left-hand side of each chart. The uppermost chart shows a portion of the gene expression profile of S. p. The lowermost chart indicates a portion of the gene expression profile of S. c.

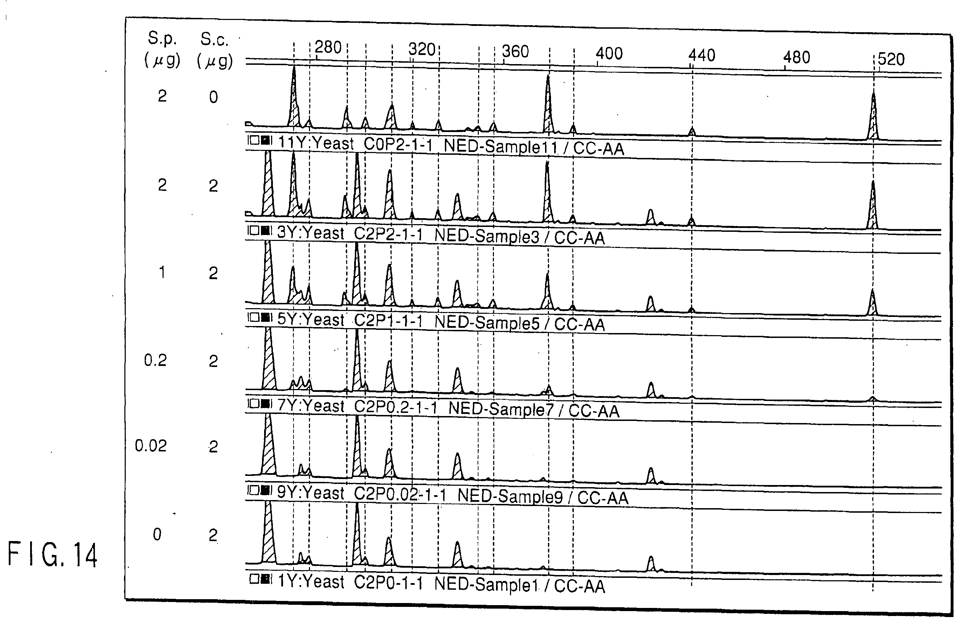

FIG. 14 is a view in which the peaks derived from S. p. in the charts of FIG. 13 are linked with vertical dotted lines. As shown in FIG. 14, the magnitude of the peaks is changed depending on the amount of mRNA of S. p. contained in the mRNA preparations.

From the results of examples 1 and 2, it has been confirmed that a gene expression profile is produced by the method of the present invention and the magnitude of expression of a gene, shown in the obtained gene expression profile, sufficiently reflects the amount of mRNA present in the sample. Accordingly, it is possible to analyze the gene expression by the method of the present invention.

According to the method of the present invention, a gene expression profile regarding genes expressed in a wide range can be produced in a simple and easy manner. Further, by using such a gene expression profile, it is possible to identity a far more number of expressed genes, i.e., substantially all of the expressed genes, as compared with the conventional method. Yet further, in the gene expression profile according to the present invention, identification of genes can be carried out for each gene. Yet further, as the gene expression profile of the present invention reflects the expression magnitude of genes, the gene expression can also be analyzed. Specifically, the expression of an unknown gene can also be analyzed as is the case with the expression of known genes.

Further advantages and modifications will easily be noticed by one skilled in the art. Therefore, in terms of the aspects of such a wide range of further advantages and modifications, the present invention is not limited to the detailed description and the representative embodiment described above. In other words, various changes may be applied to the present invention within the scope of the general idea of the invention, which is clearly shown by the accompanying claims and equivalents thereof.

## Claims

1. A method of producing a gene expression profile, comprising:
(a) a step of synthesizing cDNA from mRNA extracted from a cell, such that a tag substance is added to the 5' terminal of the cDNA;
(b) a step of cutting the product obtained as a result of the reaction in step (a) with a first restriction enzyme X;
(c) a step of connecting, to a fragment obtained in step (b), an "X" adaptor having a sequence complementary to a sequence of a site of the fragment at which site incision with the first restriction enzyme X has been effected;
(d) a step of connecting the fragment obtained in step (c) to a substance having high affinity with respect to the tag substance, thereby collecting the fragment;
(e) a step of cutting the fragment collected in step (d) with a second restriction enzyme Y and removing a fragment connected to the tag substance, thereby obtaining a fragment including the 5' side-portion of the cut cDNA;
(f) a step of adding, to a fragment obtained in step (e), a "Y" adaptor having a sequence complementary to a sequence of a site of the fragment at which site incision with the second restriction enzyme Y has been effected;
(g) a step of carrying out a PCR reaction, for the fragment obtained in step (f), by using an "X" primer which has a sequence complementary to the sequence of the "X" adaptor and two-nucleotide sequence (NN) at the 3' terminal thereof, and a "Y" primer which has a sequence complementary to the sequence of the "Y" adaptor and two-nucleotide sequence (NN) at the 3' terminal thereof; and
(h) a step of subjecting the obtained PCR product to electrophoresis and detecting a migration distance and a peak, thereby producing a gene expression profile.

2. A method of producing a gene expression profile according to claim 1, wherein the "X" primer further includes a fluorescent substance added to the 5' terminal thereof, and thereby the result of the electrophoresis of the PCR product is analyzed by detecting a magnitude of fluorescence of the fluorescence substance.

3. A method of producing a gene expression profile according to claim 1, wherein the restriction enzyme X and the restriction enzyme Y are selected, respectively, from the group consisting of the following enzymes: AccII, AfaI, AluI, AspLEI, BfaI, BscFI, Bsh1236I, BshI, BsiSI, Bsp143I BstUI, BsuRI, CfoI, Csp6I, DpnII, FnuDII, HaeIII, HapII, HhaI, Hin2I, Hin6I, HinP1I HpaII, Hsp92II, HspAI, Kzo9I, MaeI, MboI, MseI, MspI, MvnI, NdeII, NIaIII, Pall, RsaI, Sau3AI, Sse9I, TaqI, ThaI, Tru1I, Tru9I, Tsp509I, TspEI and TthHB8I.

4. A method of producing a gene expression profile according to claim 1, wherein the restriction enzyme X is MspI and the restriction enzyme Y is MseI.

5. A method of producing a gene expression profile according to claim 1, wherein the NN included in the "X" primer and the NN included in the "Y" primer are designed as a combination of adenine, thymine, guanine and cytosine, and thus totally 256 types of the "X" and "Y" primer sets are used.

6. A method of producing a gene expression profile according to claim 1, wherein combination of the tag substance and the substance having high affinity with respect to the tag substance include: biotin and streptavidin; biotin and avidin; FITC and FITC antibody; DIG and anti-DIG; protein A and mouse IgG; latex particles.

7. A method of producing a gene expression profile according to claim 1, further comprising, after step (h) of subjecting the obtained PCR product to electrophoresis and detecting a migration distance and a peak, thereby producing a gene expression profile, the step of:
collecting a molecule separated by electrophoresis and corresponding to the detected peak, and determining by sequencing the sequence of the PCR product contained therein, thereby identifying the expressed gene.

8. A method of producing a gene expression profile according to claim 1, further comprising the step of:
identifying the expressed gene, by comparing the identification sequence of the restriction enzyme X and the restriction enzyme Y, the length of the fragments produced as a result of the incision of the reaction product obtained in step (a) with the restriction enzyme X and the restriction enzyme Y, and a gene sequence data available from public data banks, with each other.

9. A method of producing a gene expression profile, comprising:
(a) a step of synthesizing cDNA from mRNA extracted from a cell, such that a tag substance is added to the 5' terminal of the cDNA, and dividing the product obtained as a result of the synthesis into two fractions;
(b) a step of cutting the first fraction of the synthesis product obtained in step (a) with a first restriction enzyme X;
(c) a step of connecting, to a fragment obtained in step (b), an "X" adaptor having a sequence complementary to a sequence of a site of the fragment at which site incision with the first restriction enzyme X has been effected;
(d) a step of connecting the fragment obtained at the step (c) to a substance having high affinity with respect to the tag substance, thereby collecting the fragment;
(e) a step of cutting the fragment collected at the step (d) with a second restriction enzyme Y and removing a fragment connected to the tag substance, thereby obtaining a fragment including the 5' side-portion of the cut cDNA;
(f) a step of adding, to a fragment obtained in step (e), a "Y" adaptor having a sequence complementary to a sequence of a site of the fragment at which site incision with the second restriction enzyme Y has been effected;
(g) a step of carrying out a PCR reaction, for the fragment obtained in step (f), by using an "X" primer which has a sequence complementary to the sequence of the "X" adaptor and a two-nucleotide sequence (NN) at the 3' terminal thereof, and a "Y" primer which has a sequence complementary to the sequence of the "Y" adaptor and a two-nucleotide sequence (NN) at the 3' terminal thereof;
(h) a step of cutting the second fraction of the synthesis product obtained in step (a) with the restriction enzyme Y;
(i) a step of connecting, to a fragment obtained in step (h), a "Y'" adaptor having a sequence complementary to a sequence of a site of the fragment at which site incision with the restriction enzyme Y has been effected;
(j) a step of connecting the fragment obtained in step (i) to a substance having high affinity with respect to the tag substance, thereby collecting the fragment;
(k) a step of cutting the fragment collected in step (j) with the restriction enzyme X and removing a fragment connected to the tag substance, thereby obtaining a fragment including the 5' side-portion of the cut cDNA;
(l) a step of adding, to a fragment obtained in step (k), an "X'" adaptor having a sequence complementary to a sequence of a site of the fragment at which site incision with the restriction enzyme X has been effected;
(m) a step of carrying out a PCR reaction, for the fragment obtained in step (1), by using a "y'" primer which has a sequence complementary to the sequence of the "Y'" adaptor and two-nucleotide sequence (NN) at the 3' terminal thereof, and an "X'" primer which has a sequence complementary to the sequence of the "X'" adaptor and two- nucleotide sequence (NN) at the 3' terminal thereof; and
(n) a step of subjecting the PCR product obtained in steps (g) and (m) to electrophoresis and detecting a migration distance and a peak, thereby producing a gene expression profile.

10. A method of producing a gene expression profile according to claim 9, wherein the "X" primer used in step (g), further includes a fluorescent substance added to the 5' terminal thereof, and
the "Y'" primer used in step (m), further includes a fluorescent substance added to the 5' terminal thereof, and the result of the electrophoresis of the PCR product is analyzed by detecting a magnitude of fluorescence of the fluorescence substance.

11. A method of producing a gene expression profile according to claim 9, wherein the restriction enzyme X and the restriction enzyme Y are selected, respectively, from the group consisting of the following enzymes: AccII, AfaI, AluI, AspLEI, BfaI, BscFI, Bsh1236I, BshI, BsiSI, Bsp143I, BstUI, BsuRI, CfoI, Csp6I, DpnII, FnuDII, HaeIII, HapII, HhaI, Hin2I, Hin6I, HinP1I, HpaII, Hsp92II, HspAI, Kzo9I, MaeI, MboI, MseI, MspI, MvnI, NdeII, N1aIII, Pall, RsaI, Sau3AI, Sse9I, TaqI, ThaI, Tru1I, Tru9I, Tsp509I, TspEI and TthHB8I.

12. A method of producing a gene expression profile according to claim 9, wherein the restriction enzyme X is MspI and the restriction enzyme Y is MseI.

13. A method of producing a gene expression profile according to claim 9, wherein the NN included in the "X" primer and the NN included in the "Y" primer are designed as a combination of adenine, thymine, guanine and cytosine, and thus totally 256 types of the "X" and "Y"primer sets are used.

14. A method of producing a gene expression profile according to claim 9, wherein combination of the tag substance and the substance having high affinity with respect to the tag substance include: biotin and streptavidin; biotin and avidin; FITC and FITC antibody; DIG and anti-DIG; protein A and mouse IgG; latex particles.

15. A method of producing a gene expression profile according to claim 9, further comprising, after step (n) of subjecting the obtained PCR product to electrophoresis and detecting a migration distance and a peak, thereby producing a gene expression profile, the step of:
collecting a molecule separated by electrophoresis and corresponding to the detected peak, and determining by sequencing the sequence of the PCR product contained therein, thereby identifying the expressed gene.

16. A method of producing a gene expression profile according to claim 9, further comprising the step of:
identifying the expressed gene, by comparing the identification sequence of the restriction enzyme X and the restriction enzyme Y, the length of the fragments produced as a result of the incision of the reaction product obtained in step (a) with the restriction enzyme X and the restriction enzyme Y, and data from any suitable data banks, with each other.

17. A method of analyzing gene expression, comprising:
(1) a step of carrying out a method of producing a gene expression profile, for each of a control cell and a subject cell, thereby producing two sets of gene expression profiles; and
(2) a step of analyzing a change in gene expression at the subject cell, by comparing the two gene expression profiles obtained in step (I),
wherein the method of producing a gene expression profile includes:
(a) a step of synthesizing cDNA from mRNA extracted from a cell, such that a tag substance is added to the 5' terminal of the cDNA;
(b) a step of cutting the product obtained as a result of the reaction in step (a) with a first restriction enzyme X;
(c) a step of connecting, to a fragment obtained in step (b), an "X" adaptor having a sequence complementary to a sequence of a site of the fragment at which site incision with the first restriction enzyme X has been effected;
(d) a step of connecting the fragment obtained in step (c) to a substance having high affinity with respect to the tag substance, thereby collecting the fragment;
(e) a step of cutting the fragment collected in step (d) with a second restriction enzyme Y and removing a fragment connected to the tag substance, thereby obtaining a fragment including the 5' side-portion of the cut cDNA;
(f) a step of adding, to a fragment obtained in step (e), a "Y" adaptor having a sequence complementary to a sequence of a site of the fragment at which site incision with the second restriction enzyme Y has been effected;
(g) a step of carrying out a PCR reaction, for the fragment obtained in step (f), by using an "X" primer which has a sequence complementary to the sequence of the "X" adaptor and two-nucleotide sequence (NN) at the 3' terminal thereof, and a "Y" primer which has a sequence complementary to the sequence of the "Y" adaptor and two-nucleotide sequence (NN) at the 3' terminal thereof; and
(h) a step of subjecting the obtained PCR product to electrophoresis and detecting a migration distance and a peak, thereby producing a gene expression profile.

18. A method of analyzing gene expression, comprising:
(1) a step of carrying out a method of producing a gene expression profile, for each of a control cell and a subject cell, thereby producing two sets of gene expression profiles; and
(2) a step of analyzing a change in gene expression at the subject cell, by comparing the two gene expression profiles obtained in step (I),
wherein the method of producing a gene expression profile includes:
(a) a step of synthesizing cDNA from mRNA extracted from a cell, such that a tag substance is added to the 5' terminal of the cDNA, and dividing the product obtained as a result of the synthesis into two fractions;
(b) a step of cutting the first fraction of the synthesis product obtained in step (a) with a first restriction enzyme X;
(c) a step of connecting, to a fragment obtained in step (b), an "X" adaptor having a sequence complementary to a sequence of a site of the fragment at which site incision with the first restriction enzyme X has been effected;
(d) a step of connecting the fragment obtained in step (c) to a substance having high affinity with respect to the tag substance, thereby collecting the fragment;
(e) a step of cutting the fragment collected in step (d) with a second restriction enzyme Y and removing a fragment connected to the tag substance, thereby obtaining a fragment including the 5' side- portion of the cut cDNA;
(f) a step of adding, to a fragment obtained in step (e), a "Y" adaptor having a sequence complementary to a sequence of a site of the fragment at which site incision with the second restriction enzyme Y has been effected;
(g) a step of carrying out a PCR reaction, for the fragment obtained in step (f), by using an "X" primer which has a sequence complementary to the sequence of the "X" adaptor and a two-nucleotide sequence (NN) at the 3' terminal thereof, and a "Y" primer which has a sequence complementary to the sequence of the "Y" adaptor and a two-nucleotide sequence (NN) at the 3' terminal thereof;
(h) a step of cutting the second fraction of the synthesis product obtained in step (a) with the restriction enzyme Y;
(i) a step of connecting, to a fragment obtained in step (h), a "Y'" adaptor having a sequence complementary to a sequence of a site of the fragment at which site incision with the restriction enzyme Y has been effected;
(j) a step of connecting the fragment obtained in step (i) to a substance having high affinity with respect to the tag substance, thereby collecting the fragment;
(k) a step of cutting the fragment collected in step (j) with the restriction enzyme X and removing a fragment connected to the tag substance, thereby obtaining a fragment including the 5' side-portion of the cut cDNA;
(l) a step of adding, to a fragment obtained in step (k), an "X'" adaptor having a sequence complementary to a sequence of a site of the fragment at which site incision with the restriction enzyme X has been effected;
(m) a step of carrying out a PCR reaction, for the fragment obtained in step (1) , by using a "Y'" primer which has a sequence complementary to the sequence of the "Y'" adaptor and two-nucleotide sequence (NN) at the 3' terminal thereof, and an "X'" primer which has a sequence complementary to the sequence of the "X'" adaptor and two-nucleotide sequence (NN) at the 3' terminal thereof; and
(n) a step of subjecting the PCR product obtained in steps (g) and (m) to electrophoresis and detecting a migration distance and a peak, thereby producing a gene expression profile.

## Patentansprüche

1. Verfahren zur Herstellung eines Genexpressionsprofils, welches Folgendes aufweist:
(a) einen Schritt des Synthetisierens von cDNA aus mRNA, welches aus einer Zelle extrahiert wurde, so dass eine Markierungssubstanz zu dem 5'-Terminal des cDNA hinzugefügt wird;
(b) einen Schritt des Schneidens des als ein Ergebnis der Reaktion in Schritt (a) erhaltenen Produkts mit einem ersten Restriktionsenzym X;
(c) einen Schritt des Verbindens eines "X"-Adapters, welcher eine Sequenz aufweist, die komplementär zu einer Sequenz eines Orts des Fragments ist, an welchem Ort der Einschnitt mit dem ersten Restriktionsenzyms X durchgeführt worden ist, mit einem in Schritt (b) erhaltenen Fragment;
(d) einen Schritt des Verbindens des in Schritt (c) erhaltenen Fragments mit einer Substanz, welche eine hohe Affinität bezüglich der Markierungssubstanz hat, wodurch das Fragment aufgenommen wird;
(e) einen Schritt des Schneidens des in Schritt (d) aufgenommenen Fragments mit einem zweiten Restriktionsenzyms Y und Entfernen eines mit der Markierungssubstanz verbundenen Fragments, wodurch ein Fragment erhalten wird, welches den 5'-Seitenabschnitt der geschnittenen cDNA beinhaltet;
(f) einen Schritt des Hinzufügens eines Y-Adapters, welcher eine Sequenz komplementär zu einer Sequenz eines Orts des Fragments aufweist, an welchem Ort der Einschnitt mit dem zweiten Restriktionsenzyms Y durchgeführt worden ist, mit einem in Schritt (e) erhaltenen Fragement;
(g) einen Schritt des Ausführens einer PCR-Reaktion für das in Schritt (f) erhaltene Fragment unter Verwendung eines "X"-Primers, welcher eine Sequenz komplementär zu der Sequenz des X-Adapters und eine Zwei-Nukleotid-Sequenz (NN) an dem 3'-Terminal desselben aufweist, und eines "Y"-Primers, welcher eine Sequenz komplementär zu der Sequenz des Y-Adapters und eine Zwei-Nukleotid-Sequenz (NN) an dem 3'-Terminal desselben aufweist; und
(h) einen Schritt des Aussetzens des erhaltenen PCR-Produkts einer Elektrophorese und Ermitteln einer Migrationsdistanz und eines Höchstwerts, wodurch ein Genexpressionsprofil hergestellt wird.

2. Verfahren zur Herstellung eines Genexpressionsprofils nach Anspruch 1, wobei der X-Primer des weiteren eine fluoreszierende Substanz aufweist, die zu dem 5'-Terminal desselben hinzugefügt wurde, und dass dadurch das Ergebnis der Elektrophorese des PCR-Produkts durch Detektieren einer Größe der Fluoreszenz der fluoriszierenden Substanz analysiert wird.

3. Verfahren zur Herstellung eines Genexpressionsprofils nach Anspruch 1, wobei das Restriktionsenzym X und das Restriktionsenzym Y jeweils aus der Gruppe ausgewählt werden, die aus den folgenden Enzymen besteht: AccII, AfaI, AluI, AspLEI, BfaI, BscFI, Bsh1236I, BshI, BsiSI, Bsp143I, BstUI, BsuRI, CfoI, Csp6I, DpnII, FnuDII, HaeIII, HapII, HhaI, Hin2I, Hin6I, HinP1I, HpaII, Hsp92II, HspAI, Kzo9I, MaeI, MboI, MseI, MspI, MvnI, NdeII, NlaIII, Pa1I, RsaI, Sau3AI, Sse9I, TagI, ThaI, Tru1I, Tru9I, Tsp509I, TspEI und TthHB8I.

4. Verfahren zur Herstellung eines Genexpressionsprofils nach Anspruch 1, wobei das Restriktionsenzym X MspI und das Restriktionsenzym Y MseI ist.

5. Verfahren zur Herstellung eines Genexpressionsprofils nach Anspruch 1, wobei das in dem "X"-Primer enthaltene NN und das in dem "Y"-Primer enthaltene NN als eine Kombination von Adenin, Thymin, Guanin und Cytosin konzipiert werden und somit insgesamt 256 Arten der "X"- und "Y"-Primersätze verwendet werden.

6. Verfahren zur Herstellung eines Genexpressionsprofils nach Anspruch 1, wobei die Kombination der Markierungssubstanz und der Substanz, welche eine hohe Affinität bezüglich der Markierungssubstanz aufweist, Folgendes beinhaltet: Biotion und Streptavidin; Biotin und Avidin; FITC und FITC-Antikörper; DIG und Anti-DIG; Protein A und Maus-IgG; Latexpartikel.

7. Verfahren zur Herstellung eines Genexpressionsprofils nach Anspruch 1, welches des weiteren nach dem Schritt (h) des Unterziehens des erhaltenen PCR-Produkts einer Elektrophorese und Detektieren einer Migrationsdistanz und eines Höchstwerts, wodurch ein Genexpressionsprofil hergestellt wird, den folgenden Schritt aufweist:
Aufnehmen eines durch Elektrophorese getrennten und dem detektierten Höchstwert entsprechenden Moleküls und Ermitteln der Sequenz des darin enthaltenen PCR-Produkts durch Sequenzieren, wodurch das exprimierte Gen identifiziert wird.

8. Verfahren zur Herstellung eines Genexpressionsprofils nach Anspruch 1, welches des weiteren den folgenden Schritt aufweist:
Identifizieren des exprimierten Gens durch Vergleichen der Identifikationssequenz des Restriktionsenzyms X und des Restriktionsenzyms Y, der Länge der als ein Ergebnis des Einschnitts des in Schritt (a) erhaltenen Reaktionsprodukts mit dem Restriktionsenzym X und dem Restriktionsenzym Y, und Gensequenzdaten, die aus öffentlichen Datenbanken zugänglich sind, miteinander.

9. Verfahren zur Herstellung eines Genexpressionsprofils, welches Folgendes aufweist:
(a) einen Schritt des Synthetisierens von cDNA aus mRNA, welches aus einer Zelle extrahiert wurde, so dass eine Markierungssubstanz zu dem 5'-Terminal des cDNA hinzugefügt wird, und Teilen des als ein Ergebnis der Synthese erhaltenen Produkts in zwei Teile;
(b) einen Schritt des Schneidens des ersten Teils des in Schritt (a) erhaltenen Syntheseprodukts mit einem ersten Restriktionsenzym X;
(c) einen Schritt des Verbindens eines "X"-Adapters, welcher eine Sequenz aufweist, die komplementär zu einer Sequenz eines Orts des Fragments ist, an welchem Ort der Einschnitt mit dem ersten Restriktionsenzyms X durchgeführt worden ist, mit einem in Schritt (b) erhaltenen Fragment;
(d) einen Schritt des Verbindens des in Schritt (c) erhaltenen Fragments mit einer Substanz, welche eine hohe Affinität bezüglich der Markierungssubstanz hat, wodurch das Fragment aufgenommen wird;
(e) einen Schritt des Schneidens des in Schritt (d) aufgenommenen Fragments mit einem zweiten Restriktionsenzyms Y und Entfernen eines mit der Markierungssubstanz verbundenen Fragments, wodurch ein Fragment erhalten wird, welches den 5'-Seitenabschnitt der geschnittenen cDNA beinhaltet;
(f) einen Schritt des Hinzufügens eines "Y"-Adapters, welcher eine Sequenz komplementär zu einer Sequenz eines Orts des Fragments aufweist, an welchem Ort der Einschnitt mit dem zweiten Restriktionsenzyms Y durchgeführt worden ist, mit einem in Schritt (e) erhaltenen Fragment;
(g) einen Schritt des Ausführens einer PCR-Reaktion für das in Schritt (f) erhaltene Fragment unter Verwendung eines "X"-Primers, welcher eine Sequenz komplementär zu der Sequenz des "X"-Adapters und eine Zwei-Nukleotid-Sequenz (NN) an dem 3'-Terminal desselben aufweist, und eines "Y"-Primers, welcher eine Sequenz komplementär zu der Sequenz des "Y"-Adapters und eine Zwei-Nukleotid-Sequenz (NN) an dem 3'-Terminal desselben aufweist;
(h) einen Schritt des Schneidens des zweiten Teils des in Schritt (a) erhaltenen Syntheseprodukts mit dem Restriktionsenzym Y;
(i) einen Schritt des Verbindens eines "Y'"-Adapters, welcher eine Sequenz aufweist, die komplementär zu einer Sequenz eines Orts des Fragments ist, an welchem Ort der Einschnitt mit dem ersten Restriktionsenzyms Y durchgeführt worden ist, mit einem in Schritt (h) erhaltenen Fragment;
(j) einen Schritt des Verbindens des in Schritt (i) erhaltenen Fragments mit einer Substanz, welche eine hohe Affinität bezüglich der Markierungssubstanz hat, wodurch das Fragment aufgenommen wird;
(k) einen Schritt des Schneidens des in Schritt (j) aufgenommenen Fragments mit einem zweiten Restriktionsenzyms X und Entfernen eines mit der Markierungssubstanz verbundenen Fragments, wodurch ein Fragment erhalten wird, welches den 5'-Seitenabschnitt der geschnittenen cDNA beinhaltet;
(l) einen Schritt des Hinzufügens eines "X'"-Adapters, welcher eine Sequenz komplementär zu einer Sequenz eines Orts des Fragments aufweist, an welchem Ort der Einschnitt mit dem zweiten Restriktionsenzyms X durchgeführt worden ist, mit einem in Schritt (k) erhaltenen Fragment;
(m) einen Schritt des Ausführens einer PCR-Reaktion für das in Schritt (l) erhaltene Fragment unter Verwendung eines "Y'"-Primers, welcher eine Sequenz komplementär zu der Sequenz des "Y'"-Adapters und eine Zwei-Nukleotid-Sequenz (NN) an dem 3'-Terminal desselben aufweist, und eines "X'"-Primers, welcher eine Sequenz komplementär zu der Sequenz des "X'"-Adapters und eine Zwei-Nukleotid-Sequenz (NN) an dem 3'-Terminal desselben aufweist; und
(n) einen Schritt des Aussetzens des in den Schritten (g) und (m) erhaltenen PCR-Produkts einer Elektrophorese und Ermitteln einer Migrationsdistanz und eines Höchstwerts, wodurch ein Genexpressionsprofil hergestellt wird.

10. Verfahren zur Herstellung eines Genexpressionsprofils nach Anspruch 9, wobei der in Schritt (g) verwendete "X"-Primer des weiteren eine fluoreszierende Substanz aufweist, die zu dem 5'-Terminal desselben hinzugefügt wurde, und der in Schritt (m) verwendete "Y'"-Primer des weiteren eine fluoreszierende Substanz aufweist, die zu dem 5'-Terminal desselben hinzugefügt wurde, und dass das Ergebnis der Elektrophorese des PCR-Produkts durch Detektiern einer Größe der Fluoreszenz der fluoreszierenden Substanz analysiert wird.

11. Verfahren zur Herstellung eines Genexpressionsprofils nach Anspruch 9, wobei das Restriktionsenzym X und das Restriktionsenzym Y jeweils aus der Gruppe ausgewählt werden, die aus den folgenden Enzymen besteht: AccII, AfaI, AluI, AspLEI, BfaI, BscFI, Bsh1236I, BshI, BsiSI, Bsp143I, BstUI, BsuRI, CfoI, Csp6I, DpnII, FnuDII, HaeIII, HapII, HhaI, Hin2I, Hin6I, HinP1I, HpaII, Hsp92II, HspAI, Kzo9I, MaeI, MboI, MseI, MspI, MvnI, NdeII, N1aIII, Pa1I, RsaI, Sau3AI, Sse9I, TagI, ThaI, Tru1I, Tru9I, Tsp509I, TspEI und TthHB8I.

12. Verfahren zur Herstellung eines Genexpressionsprofils nach Anspruch 9, wobei das Restriktionsenzym X MspI und das Restriktionsenzym Y MseI ist.

13. Verfahren zur Herstellung eines Genexpressionsprofils nach Anspruch 9, wobei das in dem "X"-Primer enthaltene NN und das in dem "Y"-Primer enthaltene NN als eine Kombination von Adenin, Thymin, Guanin und Cytosin konzipiert werden und somit insgesamt 256 Arten der "X"- und "Y"-Primersätze verwendet werden.

14. Verfahren zur Herstellung eines Genexpressionsprofils nach Anspruch 9, wobei die Kombination der Markierungssubstanz und der Substanz, welche eine hohe Affinität bezüglich der Markierungssubstanz aufweist, Folgendes beinhaltet: Biotion und Streptavidin; Biotin und Avidin; FITC und FITC-Antikörper; DIG und Anti-DIG; Protein A und Maus-IgG; Latexpartikel.

15. Verfahren zur Herstellung eines Genexpressionsprofils nach Anspruch 9, welches des weiteren nach dem Schritt (n) des Unterziehens des erhaltenen PCR-Produkts einer Elektrophorese und Detektieren einer Migrationsdistanz und eines Höchstwerts, wodurch ein Genexpressionsprofil hergestellt wird, den folgenden Schritt aufweist:
Aufnehmen eines durch Elektrophorese getrennten und dem detektierten Höchstwert entsprechenden Moleküls und Ermitteln der Sequenz des darin enthaltenen PCR-Produkts durch Sequenzieren, wodurch das exprimierte Gen identifiziert wird.

16. Verfahren zur Herstellung eines Genexpressionsprofils nach Anspruch 9, welches des weiteren den folgenden Schritt aufweist:
Identifizieren des exprimierten Gens durch Vergleichen der Identifikationssequenz des Restriktionsenzyms X und des Restriktionsenzyms Y, der Länge der als ein Ergebnis des Einschnitts des in Schritt (a) erhaltenen Reaktionsprodukts mit dem Restriktionsenzym X und dem Restriktionsenzym Y, und Daten, die aus öffentlichen Datenbanken zugänglich sind, miteinander.

17. Verfahren zur Genexpressionsanalyse, welches Folgendes aufweist:
(1) einen Schritt der Durchführung eines Verfahrens zur Herstellung eines Genexpressionsprofils, sowohl für eine Kontrollzelle als auch eine betroffene Zelle, wodurch zwei Sätze von Genexpressionsprofilen hergestellt werden; und
(2) einen Schritt der Analyse einer Veränderung in der Genexpression an der betroffenen Zelle durch Vergleichen der zwei in Schritt (1) erhaltenen Genexpressionsprofile,
wobei das Verfahren zur Herstellung eines Genexpressionsprofils Folgendes aufweist:
(a) einen Schritt des Synthetisierens von cDNA aus mRNA, welches aus einer Zelle extrahiert wurde, so dass eine Markierungssubstanz zu dem 5'-Terminal des cDNA hinzugefügt wird;
(b) einen Schritt des Schneidens des als ein Ergebnis der Reaktion in Schritt (a) erhaltenen Produkts mit einem ersten Restriktionsenzym X;
(c) einen Schritt des Verbindens eines "X"-Adapters, welcher eine Sequenz aufweist, die komplementär zu einer Sequenz eines Orts des Fragments ist, an welchem Ort der Einschnitt mit dem ersten Restriktionsenzyms X durchgeführt worden ist, mit einem in Schritt (b) erhaltenen Fragment;
(d) einen Schritt des Verbindens des in Schritt (c) erhaltenen Fragments mit einer Substanz, welche eine hohe Affinität bezüglich der Markierungssubstanz hat, wodurch das Fragment aufgenommen wird;
(e) einen Schritt des Schneidens des in Schritt (d) aufgenommenen Fragments mit einem zweiten Restriktionsenzyms Y und Entfernen eines mit der Markierungssubstanz verbundenen Fragments, wodurch ein Fragment erhalten wird, welches den 5'-Seitenabschnitt der geschnittenen cDNA beinhaltet;
(f) einen Schritt des Hinzufügens eines "Y"-Adapters, welcher eine Sequenz komplementär zu einer Sequenz eines Orts des Fragments aufweist, an welchem Ort der Einschnitt mit dem zweiten Restriktionsenzyms Y durchgeführt worden ist, mit einem in Schritt (e) erhaltenen Fragement;
(g) einen Schritt des Ausführens einer PCR-Reaktion für das in Schritt (f) erhaltene Fragment unter Verwendung eines "X"-Primers, welcher eine Sequenz komplementär zu der Sequenz des "X"-Adapters und eine Zwei-Nukleotid-Sequenz (NN) an dem 3'-Terminal desselben aufweist, und eines "Y"-Primers, welcher eine Sequenz komplementär zu der Sequenz des "Y"-Adapters und eine Zwei-Nukleotid-Sequenz (NN) an dem 3'-Terminal desselben aufweist; und
(h) einen Schritt des Aussetzens des erhaltenen PCR-Produkts einer Elektrophorese und Ermitteln einer Migrationsdistanz und eines Höchstwerts, wodurch ein Genexpressionsprofil hergestellt wird.

18. Verfahren zur Genexpressionsanalyse, welches Folgendes aufweist:
(1) einen Schritt der Durchführung eines Verfahrens zur Herstellung eines Genexpressionsprofils, sowohl für eine Kontrollzelle als auch eine betroffene Zelle, wodurch zwei Sätze von Genexpressionsprofilen hergestellt werden; und
(2) einen Schritt der Analyse einer Veränderung in der Genexpression an der betroffenen Zelle durch Vergleichen der zwei in Schritt (1) erhaltenen Genexpressionsprofile,
wobei das Verfahren zur Herstellung eines Genexpressionsprofils Folgendes aufweist:
(a) einen Schritt des Synthetisierens von cDNA aus mRNA, welches aus einer Zelle extrahiert wurde, so dass eine Markierungssubstanz zu dem 5'-Terminal des cDNA hinzugefügt wird, und Teilen des als ein Ergebnis der Synthese erhaltenen Produkts in zwei Teile;
(b) einen Schritt des Schneidens des ersten Teils des in Schritt (a) erhaltenen Syntheseprodukts mit einem ersten Restriktionsenzym X;
(c) einen Schritt des Verbindens eines "X"-Adapters, welcher eine Sequenz aufweist, die komplementär zu einer Sequenz eines Orts des Fragments ist, an welchem Ort der Einschnitt mit dem ersten Restriktionsenzyms X durchgeführt worden ist, mit einem in Schritt (b) erhaltenen Fragment;
(d) einen Schritt des Verbindens des in Schritt (c) erhaltenen Fragments mit einer Substanz, welche eine hohe Affinität bezüglich der Markierungssubstanz hat, wodurch das Fragment aufgenommen wird;
(e) einen Schritt des Schneidens des in Schritt (d) aufgenommenen Fragments mit einem zweiten Restriktionsenzyms Y und Entfernen eines mit der Markierungssubstanz verbundenen Fragments, wodurch ein Fragment erhalten wird, welches den 5'-Seitenabschnitt der geschnittenen cDNA beinhaltet;
(f) einen Schritt des Hinzufügens eines "Y"-Adapters, welcher eine Sequenz komplementär zu einer Sequenz eines Orts des Fragments aufweist, an welchem Ort der Einschnitt mit dem zweiten Restriktionsenzyms Y durchgeführt worden ist, mit einem in Schritt (e) erhaltenen Fragment;
(g) einen Schritt des Ausführens einer PCR-Reaktion für das in Schritt (f) erhaltene Fragment unter Verwendung eines "X"-Primers, welcher eine Sequenz komplementär zu der Sequenz des "X"-Adapters und eine Zwei-Nukleotid-Sequenz (NN) an dem 3'-Terminal desselben aufweist, und eines "Y"-Primers, welcher eine Sequenz komplementär zu der Sequenz des "Y"-Adapters und eine Zwei-Nukleotid-Sequenz (NN) an dem 3'-Terminal desselben aufweist;
(h) einen Schritt des Schneidens des zweiten Teils des in Schritt (a) erhaltenen Syntheseprodukts mit dem Restriktionsenzym Y;
(i) einen Schritt des Verbindens eines "Y'"-Adapters, welcher eine Sequenz aufweist, die komplementär zu einer Sequenz eines Orts des Fragments ist, an welchem Ort der Einschnitt mit dem ersten Restriktionsenzyms Y durchgeführt worden ist, mit einem in Schritt (h) erhaltenen Fragment;
(j) einen Schritt des Verbindens des in Schritt (i) erhaltenen Fragments mit einer Substanz, welche eine hohe Affinität bezüglich der Markierungssubstanz hat, wodurch das Fragment aufgenommen wird;
(k) einen Schritt des Schneidens des in Schritt (j) aufgenommenen Fragments mit einem zweiten Restriktionsenzyms X und Entfernen eines mit der Markierungssubstanz verbundenen Fragments, wodurch ein Fragment erhalten wird, welches den 5'-Seitenabschnitt der geschnittenen cDNA beinhaltet;
(l) einen Schritt des Hinzufügens eines "X'"-Adapters, welcher eine Sequenz komplementär zu einer Sequenz eines Orts des Fragments aufweist, an welchem Ort der Einschnitt mit dem zweiten Restriktionsenzyms X durchgeführt worden ist, mit einem in Schritt (k) erhaltenen Fragment;
(m) einen Schritt des Ausführens einer PCR-Reaktion für das in Schritt (l) erhaltene Fragment unter Verwendung eines "Y'"-Primers, welcher eine Sequenz komplementär zu der Sequenz des "Y'"-Adapters und eine Zwei-Nukleotid-Sequenz (NN) an dem 3'-Terminal desselben aufweist, und eines "X'"-Primers, welcher eine Sequenz komplementär zu der Sequenz des "X'"-Adapters und eine Zwei-Nukleotid-Sequenz (NN) an dem 3'-Terminal desselben aufweist; und
(n) einen Schritt des Aussetzens des in den Schritten (g) und (m) erhaltenen PCR-Produkts einer Elektrophorese und Ermitteln einer Migrationsdistanz und eines Höchstwerts, wodurch ein Genexpressionsprofil hergestellt wird.

## Revendications

1. Procédé de production d'un profil d'expression génétique, comprenant :
(a) une étape de synthèse d'un ADNc à partir d'ARNm extrait d'une cellule, de sorte qu'une substance étiquette est ajoutée à l'extrémité 5' de l'ADNc ;
(b) une étape de découpe du produit obtenu en résultat de la réaction à l'étape (a) avec une première enzyme de restriction X ;
(c) une étape de connexion, à un fragment obtenu à l'étape (b), d'un adaptateur « X » ayant une séquence complémentaire d'une séquence d'un site du fragment, site au niveau duquel une incision avec la première enzyme de restriction X a été effectuée ;
(d) une étape de connexion du fragment obtenu à l'étape (c) à une substance ayant une forte affinité à l'égard de la substance étiquette, recueillant ainsi le fragment ;
(e) une étape de découpe du fragment recueilli à l'étape (d) avec une deuxième enzyme de restriction Y et l'élimination d'un fragment relié à la substance étiquette, obtenant ainsi un fragment incluant la partie du côté 5' de l'ADNc coupé ;
(f) une étape d'ajout, à un fragment obtenu à l'étape (e), d'un adaptateur « Y » ayant une séquence complémentaire d'une séquence d'un site du fragment, site au niveau duquel une incision avec la deuxième enzyme de restriction Y a été effectuée ;
(g) une étape de réalisation d'une réaction de PCR, pour le fragment obtenu à l'étape (f), en utilisant une amorce « X » qui a une séquence complémentaire de la séquence de l'adaptateur « X » et une séquence à deux nucléotides (NN) à l'extrémité 3' de celle-ci, et une amorce « Y » qui a une séquence complémentaire de la séquence de l'adaptateur « Y » et une séquence à deux nucléotides (NN) à l'extrémité 3' de celle-ci ; et
(h) une étape de soumission du produit de PCR obtenu à une électrophorèse et de détection d'une distance de migration et d'un pic, produisant ainsi un profil d'expression génétique.

2. Procédé de production d'un profil d'expression génétique selon la revendication 1, dans lequel l'amorce « X » inclut en outre une substance fluorescente ajoutée à l'extrémité 5' de celle-ci, et ainsi le résultat de l'électrophorèse du produit de PCR est analysé par la détection d'une amplitude de fluorescence de la substance fluorescente.

3. Procédé de production d'un profil d'expression génétique selon la revendication 1, dans lequel l'enzyme de restriction X et l'enzyme de restriction y sont choisies, respectivement, dans le groupe constitué des enzymes suivantes : AccII, AfaI, AluI, AspLEI, BfaI, BscFI, Bsh1236I, BshI, BsiSI, Bsp143I, BstUI, BsuRI, CfoI, Csp6I, DpnII, FnuDII, HaeIII, HapII, HhaI, Hin2I, Hin6I, HinP1I, HpaII, Hsp92II, HspAI, Kzo9I, MaeI, MboI, MseI, MspI, MvnI, NdeII, N1aIII, Pa1I, RsaI, Sau3AI, Sse9I, TaqI, ThaI, Tru1I, Tru9I, Tsp509I, TspEI et TthHB8I.

4. Procédé de production d'un profil d'expression génétique selon la revendication 1, dans lequel l'enzyme de restriction X est MspI et l'enzyme de restriction Y est MseI.

5. Procédé de production d'un profil d'expression génétique selon la revendication 1, dans lequel la NN incluse dans l'amorce « X » et la NN incluse dans l'amorce « Y » sont conçues sous la forme d'une combinaison d'adénine, de thymine, de guanine et de cytosine, et ainsi 256 types des jeux d'amorces « X » et « Y » sont utilisés au total.

6. Procédé de production d'un profil d'expression génétique selon la revendication 1, dans lequel une combinaison de la substance étiquette et de la substance ayant une forte affinité à l'égard de la substance étiquette inclut : la biotine et la streptavidine ; la biotine et l'avidine ; la FITC et un anticorps FITC ; la DIG et un anti-DIG ; la protéine A et une IgG de souris ; des particules de latex.

7. Procédé de production d'un profil d'expression génétique selon la revendication 1, comprenant en outre, après l'étape (h) de soumission du produit de PCR obtenu à une électrophorèse et de détection d'une distance de migration et d'un pic, produisant ainsi un profil d'expression génétique, l'étape consistant à :
recueillir une molécule séparée par électrophorèse et correspondant au pic détecté, et déterminer par un séquençage la séquence du produit de PCR contenu dans celle-ci, identifiant ainsi le gène exprimé.

8. Procédé de production d'un profil d'expression génétique selon la revendication 1, comprenant en outre l'étape consistant à :
identifier le gène exprimé, en comparant la séquence d'identification de l'enzyme de restriction X et l'enzyme de restriction Y, la longueur des fragments produits en résultat de l'incision du produit réactionnel obtenu à l'étape (a) avec l'enzyme de restriction X et l'enzyme de restriction Y, et une donnée de séquence génétique accessible depuis des banques de données publiques, les unes avec les autres.

9. Procédé de production d'un profil d'expression génétique, comprenant :
(a) une étape de synthèse d'un ADNc à partir d'ARNm extrait d'une cellule, de sorte qu'une substance étiquette est ajoutée à l'extrémité 5' de l'ADNc, et de division du produit obtenu en résultat de la synthèse en deux fractions ;
(b) une étape de découpe de la première fraction du produit de synthèse obtenu à l'étape (a) avec une première enzyme de restriction X ;
(c) une étape de connexion, à un fragment obtenu à l'étape (b), d'un adaptateur « X » ayant une séquence complémentaire de la séquence d'un site du fragment, site au niveau duquel une incision avec la première enzyme de restriction X a été effectuée ;
(d) une étape de connexion du fragment obtenu à l'étape (c) à une substance ayant une forte affinité à l'égard de la substance étiquette, recueillant ainsi le fragment ;
(e) une étape de découpe du fragment recueilli à l'étape (d) avec une deuxième enzyme de restriction Y et d'élimination d'un fragment relié à la substance étiquette, obtenant ainsi un fragment incluant la partie du côté 5' de l'ADNc coupé ;
(f) une étape d'ajout, à un fragment obtenu à l'étape (e), d'un adaptateur « Y » ayant une séquence complémentaire d'une séquence d'un site du fragment, site au niveau duquel une incision avec la deuxième enzyme de restriction Y a été effectuée ;
(g) une étape de réalisation d'une réaction de PCR, pour le fragment obtenu à l'étape (f), en utilisant une amorce « X » qui a une séquence complémentaire de la séquence de l'adaptateur « X » et une séquence à deux nucléotides (NN) à l'extrémité 3' de celle-ci, et une amorce « Y » qui a une séquence complémentaire de la séquence de l'adaptateur « Y » et une séquence à deux nucléotides (NN) à l'extrémité 3' de celle-ci ;
(h) une étape de découpe de la deuxième fraction du produit de synthèse obtenu à l'étape (a) avec l'enzyme de restriction Y ;
(i) une étape de connexion, à un fragment obtenu à l'étape (h), d'un adaptateur « Y' » ayant une séquence complémentaire d'une séquence d'un site du fragment, site au niveau duquel une incision avec l'enzyme de restriction Y a été effectuée ;
(j) une étape de connexion du fragment obtenu à l'étape (i) à une substance ayant une forte affinité à l'égard de la substance étiquette, recueillant ainsi le fragment ;
(k) une étape de découpe du fragment recueilli à l'étape (j) avec l'enzyme de restriction X et d'élimination d'un fragment relié à la substance étiquette, obtenant ainsi un fragment incluant la partie du côté 5' de l'ADNc coupé ;
(l) une étape d'ajout, à un fragment obtenu à l'étape (k), d'un adaptateur «X'» ayant une séquence complémentaire d'une séquence d'un site du fragment, site au niveau duquel une incision avec l'enzyme de restriction X a été effectuée ;
(m) une étape de réalisation d'une réaction de PCR, pour le fragment obtenu à l'étape (1), en utilisant une amorce « Y ' » qui a une séquence complémentaire de la séquence de l'adaptateur « Y' » et une séquence à deux nucléotides (NN) à l'extrémité 3' de celle-ci, et une amorce « X ' » qui a une séquence complémentaire de la séquence de l'adaptateur « X' » et une séquence à deux nucléotides (NN) à l'extrémité 3' de celle-ci ; et
(n) une étape de soumission du produit de PCR obtenu aux étapes (g) et (m) à une électrophorèse et de détection d'une distance de migration et d'un pic, produisant ainsi un profil d'expression génétique.

10. Procédé de production d'un profil d'expression génétique selon la revendication 9, dans lequel l'amorce « X » utilisée à l'étape (g), inclut en outre une substance fluorescente ajoutée à l'extrémité 5' de celle-ci, et
l'amorce « Y' » utilisée à l'étape (m), inclut en outre une substance fluorescente ajoutée à l'extrémité 5' de celle-ci, et le résultat de l'électrophorèse du produit de PCR est analysé par la détection d'une amplitude de fluorescence de la substance fluorescente.

11. Procédé de production d'un profil d'expression génétique selon la revendication 9, dans lequel l'enzyme de restriction X et l'enzyme de restriction y sont choisies, respectivement, dans le groupe constitué des enzymes suivantes : AccII, AfaI, AluI, AspLEI, BfaI, BscFI, Bsh1236I, BshI, BsiSI, Bsp143I, BstUI, BsuRI, CfoI, Csp6I, DpnII, FnuDII, HaeIII, HapII, HhaI, Hin2I, Hin6I, HinP1I, HpaII, Hsp92II, HspAI, Kzo9I, MaeI, MboI, MseI, MspI, MvnI, NdeII, N1aIII, Pa1I, RsaI, Sau3AI, Sse9I, TaqI, ThaI, Tru1I, Tru9I, Tsp509I, TspEI et TthHB8I.

12. Procédé de production d'un profil d'expression génétique selon la revendication 9, dans lequel l'enzyme de restriction X est MspI et l'enzyme de restriction Y est MseI.

13. Procédé de production d'un profil d'expression génétique selon la revendication 9, dans lequel la NN incluse dans l'amorce « X » et la NN incluse dans l'amorce « Y » sont conçues sous la forme d'une combinaison d'adénine, de thymine, de guanine et de cytosine, et ainsi 256 types des jeux d'amorces « X » et « Y » sont utilisés au total.

14. Procédé de production d'un profil d'expression génétique selon la revendication 9, dans lequel une combinaison de la substance étiquette et de la substance ayant une forte affinité à l'égard de la substance étiquette inclut : la biotine et la streptavidine ; la biotine et l'avidine ; la FITC et un anticorps FITC ; la DIG et un anti-DIG ; la protéine A et une IgG de souris ; des particules de latex.

15. Procédé de production d'un profil d'expression génétique selon la revendication 9, comprenant en outre, après l'étape (n) de soumission du produit de PCR obtenu à une électrophorèse et de détection d'une distance de migration et d'un pic, produisant ainsi un profil d'expression génétique, l'étape consistant à :
recueillir une molécule séparée par électrophorèse et correspondant au pic détecté, et déterminer par un séquençage la séquence du produit de PCR contenu dans celle-ci, identifiant ainsi le gène exprimé.

16. Procédé de production d'un profil d'expression génétique selon la revendication 9, comprenant en outre l'étape consistant à :
identifier le gène exprimé, en comparant la séquence d'identification de l'enzyme de restriction X et l'enzyme de restriction Y, la longueur des fragments produits en résultat de l'incision du produit réactionnel obtenu à l'étape (a) avec l'enzyme de restriction X et l'enzyme de restriction Y, et une(des) donnée(s) provenant de n'importe quelle banque de données appropriée, les unes avec les autres.

17. Procédé d'analyse de l'expression génétique, comprenant :
(1) une étape de réalisation d'un procédé de production d'un profil d'expression génétique, pour chacune d'une cellule témoin et d'une cellule sujet, produisant ainsi deux jeux de profils d'expression génétique ; et
(2) une étape d'analyse d'un changement dans l'expression génétique au niveau de la cellule sujet, par comparaison des deux profils d'expression génétique obtenus à l'étape (I),
dans lequel le procédé de production d'un profil d'expression génétique inclut :
(a) une étape de synthèse d'un ADNc à partir d'ARNm extrait d'une cellule, de sorte qu'une substance étiquette est ajoutée à l'extrémité 5' de l'ADNc ;
(b) une étape de découpe du produit obtenu en résultat de la réaction à l'étape (a) avec une première enzyme de restriction X ;
(c) une étape de connexion, à un fragment obtenu à l'étape (b), d'un adaptateur « X » ayant une séquence complémentaire d'une séquence d'un site du fragment, site au niveau duquel une incision avec la première enzyme de restriction X a été effectuée ;
(d) une étape de connexion du fragment obtenu à l'étape (c) à une substance ayant une forte affinité à l'égard de la substance étiquette, recueillant ainsi le fragment ;
(e) une étape de découpe du fragment recueilli à l'étape (d) avec une deuxième enzyme de restriction Y et l'élimination d'un fragment relié à la substance étiquette, obtenant ainsi un fragment incluant la partie du côté 5' de l'ADNc coupé ;
(f) une étape d'ajout, à un fragment obtenu à l'étape (e), d'un adaptateur « Y » ayant une séquence complémentaire d'une séquence d'un site du fragment, site au niveau duquel une incision avec la deuxième enzyme de restriction Y a été effectuée ;
(g) une étape de réalisation d'une réaction de PCR, pour le fragment obtenu à l'étape (f), en utilisant une amorce « X » qui a une séquence complémentaire de la séquence de l'adaptateur « X » et une séquence à deux nucléotides (NN) à l'extrémité 3' de celle-ci, et une amorce « Y » qui a une séquence complémentaire de la séquence de l'adaptateur « Y » et une séquence à deux nucléotides (NN) à l'extrémité 3' de celle-ci ; et
(h) une étape de soumission du produit de PCR obtenu à une électrophorèse et de détection d'une distance de migration et d'un pic, produisant ainsi un profil d'expression génétique.

18. Procédé d'analyse de l'expression génétique, comprenant :
(1) une étape de réalisation d'un procédé de production d'un profil d'expression génétique, pour chacune d'une cellule témoin et d'une cellule sujet, produisant ainsi deux jeux de profils d'expression génétique ; et
(2) une étape d'analyse d'un changement dans l'expression génétique au niveau de la cellule sujet, par comparaison des deux profils d'expression génétique obtenus à l'étape (I),
dans lequel le procédé de production d'un profil d'expression génétique inclut :
(a) une étape de synthèse d'un ADNc à partir d'ARNm extrait d'une cellule, de sorte qu'une substance étiquette est ajoutée à l'extrémité 5' de l'ADNc, et de division du produit obtenu en résultat de la synthèse en deux fractions ;
(b) une étape de découpe de la première fraction du produit de synthèse obtenu à l'étape (a) avec une première enzyme de restriction X ;
(c) une étape de connexion, à un fragment obtenu à l'étape (b), d'un adaptateur « X » ayant une séquence complémentaire de la séquence d'un site du fragment, site au niveau duquel une incision avec la première enzyme de restriction X a été effectuée ;
(d) une étape de connexion du fragment obtenu à l'étape (c) à une substance ayant une forte affinité à l'égard de la substance étiquette, recueillant ainsi le fragment ;
(e) une étape de découpe du fragment recueilli à l'étape (d) avec une deuxième enzyme de restriction Y et d'élimination d'un fragment relié à la substance étiquette, obtenant ainsi un fragment incluant la partie du côté 5' de l'ADNc coupé ;
(f) une étape d'ajout, à un fragment obtenu à l'étape (e), d'un adaptateur « Y » ayant une séquence complémentaire d'une séquence d'un site du fragment, site au niveau duquel une incision avec la deuxième enzyme de restriction Y a été effectuée ;
(g) une étape de réalisation d'une réaction de PCR, pour le fragment obtenu à l'étape (f), en utilisant une amorce « X » qui a une séquence complémentaire de la séquence de l'adaptateur « X » et une séquence à deux nucléotides (NN) à l'extrémité 3' de celle-ci, et une amorce « Y » qui a une séquence complémentaire de la séquence de l'adaptateur « Y » et une séquence à deux nucléotides (NN) à l'extrémité 3' de celle-ci ;
(h) une étape de découpe de la deuxième fraction du produit de synthèse obtenu à l'étape (a) avec l'enzyme de restriction Y ;
(i) une étape de connexion, à un fragment obtenu à l'étape (h), d'un adaptateur « Y' » ayant une séquence complémentaire d'une séquence d'un site du fragment, site au niveau duquel une incision avec l'enzyme de restriction Y a été effectuée ;
(j) une étape de connexion du fragment obtenu à l'étape (i) à une substance ayant une forte affinité à l'égard de la substance étiquette, recueillant ainsi le fragment ;
(k) une étape de découpe du fragment recueilli à l'étape (j) avec l'enzyme de restriction X et d'élimination d'un fragment relié à la substance étiquette, obtenant ainsi un fragment incluant la partie du côté 5' de l'ADNc coupé ;
(l) une étape d'ajout, à un fragment obtenu à l'étape (k), d'un adaptateur «X'» ayant une séquence complémentaire d'une séquence d'un site du fragment, site au niveau duquel une incision avec l'enzyme de restriction X a été effectuée ;
(m) une étape de réalisation d'une réaction de PCR, pour le fragment obtenu à l'étape (1), en utilisant une amorce « Y' » qui a une séquence complémentaire de la séquence de l'adaptateur « Y' » et une séquence à deux nucléotides (NN) à l'extrémité 3' de celle-ci, et une amorce « X' » qui a une séquence complémentaire de la séquence de l'adaptateur « X' » et une séquence à deux nucléotides (NN) à l'extrémité 3' de celle-ci ; et
(n) une étape de soumission du produit de PCR obtenu aux étapes (g) et (m) à une électrophorèse et de détection d'une distance de migration et d'un pic, produisant ainsi un profil d'expression génétique.


### SEQUENCE LISTING

<110> AISIN SEIKI KABUSHIKI KAISHA
   National Institute of Radiological Sciences
   Maze, Inc.
   ABE, Masumi
   SAITO, Toshiyuki
   HATTORI, Atsushi
   SATO, Shinji
   KASAMA, Koji
<120> Method for analysing of gene expression
<130> 01S1459P
<150> JP 2000-377887
   <151> 2000-12-12
<160> 24
<170> PatentIn version 3.1
<210> 1
   <211> 22
   <212> DNA
   <213> Artificial
<400> 1
   cgggtcgtat cagacttgca ca 22
<210> 2
   <211> 20
   <212> DNA
   <213> Artificial
<400> 2
   tgtgcaagtc tgatacgacc 20
<210> 3
   <211> 22
   <212> DNA
   <213> Artificial
<400> 3
   tacatcaggt gtccgatgat tc 22
<210> 4
   <211> 20
   <212> DNA
   <213> Artificial
<400> 4
   gaatcatcgg acacctgatg 20
<210> 5
   <211> 22
   <212> DNA
   <213> Artificial
<400> 5 22
   cgagtcgtat cagacttgca ca 22
<210> 6
   <211> 20
   <212> DNA
   <213> Artificial
<400> 6 20
   tgtgcaagtc tgatacgact 20
<210> 7
   <211> 22
   <212> DNA
   <213> Artificial
<400> 7 12
   tacttggact acagtcgtga ca 22
<210> 8
   <211> 20
   <212> DNA
   <213> Artificial
<400> 8 20
   tgtcacgact gtagtccaag 20
<210> 9
<211> 116
   <212> DNA
   <213> Homo sapiens
<400> 9
   gaaaccgggg tgggtggtgt ctccaggtca atcaacttct gtactgggct ctgaccacaa 60
   tcggttttca gaccacaatg ttaggagggt atttttacat ccctccagtt aacaaa 116
<210> 10
   <211> 116
   <212> DNA
   <213> Homo sapiens
<400> 10
   gaaaccgggg tgggtggtgt ctccaggtca atcaacttct gtactgggct ctgaccacaa 60
   tcggttttca gaccacaatg ttaggagggt atttttacat ccctccagtt aacaaa 116
<210> 11
   <211> 116
   <212> DNA
   <213> Homo sapiens
<400> 11
   gaaaccgggg tgggtggtgt ctccaggtca atcaacttct gtactgggct ctgaccacaa 60
   tcggttttca gaccacaatg ttaggagggt atttttacat ccctccagtt aacaaa 116
<210> 12
   <211> 116
   <212> DNA
   <213> Homo sapiens
<400> 12
   gaaaccgggg tgggtggtgt ctccaggtca atcaacttct gtactgggct ctgaccacaa 60
   tcggttttca gaccacaatg ttaggagggt atttttacat ccctccagtt aacaaa 116
<210> 13
   <211> 116
   <212> DNA
   <213> Homo sapiens
<400> 13
   gaaaccgggg tgggtggtgt ctccaggtca atcaacttct gtactgggct ctgaccacaa 60
   tcggttttca gaccacaatg ttaggagggt atttttacat ccctccagtt aacaaa 116
<210> 14
   <211> 116
   <212> DNA
   <213> Homo sapiens
<400> 14
   gaaaccgggg tgggtggtgt ctccaggtca atcaacttct gtactgggct ctgaccacaa 60
   tcggttttca gaccacaatg ttaggagggt atttttacat ccctccagtt aacaaa 116
<210> 15
   <211> 116
   <212> DNA
   <213> Homo sapiens
<400> 15
   gaaaccgggg tgggtggtgt ctccaggtca atcaacttct gtactgggct ctgaccacaa 60
   tcggttttca gaccacaatg ttaggagggt atttttacat ccctccagtt aacaaa 116
<210> 16
   <211> 116
   <212> DNA
   <213> Homo sapiens
<400> 16
   gaaaccgggg tgggtggtgt ctccaggtca atcaacttct gtactgggct ctgaccacaa 60
   tcggttttca gaccacaatg ttaggagggt atttttatat ccctccagtt aacaaa 116
<210> 17
   <211> 116
   <212> DNA
   <213> Homo sapiens
<400> 17
   gaaaccgggg tgggtggtgt ctccaggtca atcaacttct gtactgggct ctgaccacaa 60
   tcggttttca gaccacaatg ttaggagggt atttttatat ccctccagtt aacaaa 116
<210> 18
   <211> 116
   <212> DNA
   <213> Homo sapiens
<400> 18
   gaaaccgggg tgggtggtgt ctccaggtca atcaacttct gtactgggct ctgaccacaa 60
   tcggttttca gaccacaatg ttaggagggt atttttacat ccctccagtt aacaaa 116
<210> 19
   <211> 116
   <212> DNA
   <213> Homo sapiens
<400> 19
   gaaaccaggg tgggtggtgt ctccaggtca atcaacttct gtactgggct ctgaccacaa 60
   tcggttttca gaccacaatg ttaggagggt atttttacat ccctccagtt aacaaa 116
<210> 20
   <211> 116
   <212> DNA
   <213> Homo sapiens
<400> 20
   gaaaccgggg tgggtggtgt ctccaggtca atcaacttct gtactgggct ctgaccacaa 60
   tcggttttca gaccacaatg ttaggagggt atttttacat ccctccagtt aacaaa 116
<210> 21
   <211> 116
   <212> DNA
   <213> Homo sapiens
<400> 21
   gaaaccgggg tgggtggtgt ctccaggtca atcaacttct gtactgggct ctgaccacaa 60
   tcggttttca gaccacaatg ttaggagggt atttttatat ccctccagtt aacaaa 116
<210> 22
   <211> 116
   <212> DNA
   <213> Homo sapiens
<400> 22
   gaaaccgggg tgggtggtgt ctccaggtca atcaacttct gtactgggct ctgaccacaa 60
   tcggttttca gaccacaatg ttaggagggt atttttacat ccctccagtt aacaaa 116
<210> 23
   <211> 116
   <212> DNA
   <213> Homo sapiens
<400> 23
   gaaaccgggg tgggtggtgt ctccaggtca atcaacttct gtactgggct ctgaccacaa 60
   tcggttttca gaccacaatg ttaggagggt atttttacat ccctccagtt aacaaa 116
<210> 24
   <211> 116
   <212> DNA
   <213> Homo sapiens
<400> 24
   gaaaccaggg tgggtggtgt ctccaggtca atcaacttct gtactgggct ctgaccacaa 60
   tcggttttca gaccacaatg ttaggagggt atttttatat ccctccagtt aacaaa 116